(19) Europäisches Patentamt
European Patent Office
Office européen des brevets

(11) Publication number: **0 536 424 A1**

(12) **EUROPEAN PATENT APPLICATION published in accordance with Art. 158(3) EPC**

(21) Application number: **92909551.1**

(22) Date of filing: **24.04.92**

(86) International application number: **PCT/JP92/00538**

(87) International publication number: **WO 92/19611 (12.11.92 92/28)**

(51) Int. Cl.[5]: **C07D 311/68**, C07D 405/12, C07D 409/12, //A61K31/35, (C07D405/12,213:00,311:00), (C07D409/12,311:00,333:00)

(30) Priority: **26.04.91 JP 188374/91**
**30.07.91 JP 279014/91**
**15.04.92 JP 137484/92**

(43) Date of publication of application: **14.04.93 Bulletin 93/15**

(84) Designated Contracting States: **AT BE CH DE DK ES FR GB GR IT LI LU MC NL SE**

(71) Applicant: **Japan Tobacco Inc.**
**4-12-62 Higashishinagawa**
**Shinagawa-ku, Tokyo 140(JP)**

(72) Inventor: **KATOH, Susumu, Pharmaceutical Research Laboratory**
**of Japan Tobacco Inc, 6-2, Umegaoka, Midori-ku**
**Yokohama-shi, Kanagawa-ken 227(JP)**
Inventor: **SAYAMA, Shinsuke, Pharmaceutical Research Labor.**
**of Japan Tobacco Inc., 6-2, Umegaoka, Modori-ku**
**Yokohama-shi, Kanagawa-ken 227(JP)**
Inventor: **SHIBATA, Saizo, Pharmaceutical Research Laboratory**
**of Japan Tobacco Inc., 6-2, Umegaoka, Modori-ku**
**Yokohama-shi, Kanagawa-ken 227(JP)**
Inventor: **UCHIDA, Itsuo, Pharmaceutical Research Laboratory**
**of Japan Tobacco Inc., 6-2, Umegaoka, Modori-ku**
**Yokohama-shi, Kanagawa-ken 227(JP)**
Inventor: **YAMAKI, Tokuo, Toxicology Research Laboratory of**
**Japan Tobacco inc., 23, Nakogi**
**Hadano-shi, Kanagawa-ken 257(JP)**

(74) Representative: **Reinhard, Horst (DE) et al**
**Reinhard-Skuhra-Weise Friedrichstrasse 31**
**W-8000 München 40 (DE)**

(54) **NOVEL BENZOPYRAN DERIVATIVE.**

(57) A novel benzopyran derivative represented by general formula (I) and a pharmaceutically acceptable salt thereof, wherein each symbol is as defined in the specification. This compound is useful for treating hypertension, because it has a persistent hypotensive activity and a peripheral blood relaxing activity by activating a potassium channel, and also for treating cardiovascular diseases such as angina pectoris and heart failure, because it has a selective coronary blood stream increasing activity.

EP 0 536 424 A1

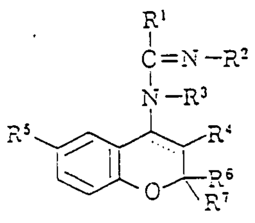

(I)

[Technical Field]

The present invention relates to novel benzopyran derivatives useful as pharmaceutial substances, which permit activating a potassium channel and relax smooth muscles such as a blood vessel smooth muscle.

[Background Art]

A potassium channel relates to a resting membrane potential. If the potassium channel is activated, the resting membrane potential is shifted in the negative direction (hyper-polarization) and approaches the equilibrium potential of potassium ions. Also, activation of the potassium channel inhibits the activation of a potential-dependent calcium channel so as to suppress the calcium influx. At the same time, a sodium-calcium exchange reaction is promoted so as to facilitate release of intracellular calcium from within the cells. The hyper-polarization of the membrane and the subsequent reduction in the free calcium concentration within the cell permit relaxing smooth muscles, resulting the vasodilation so as to lower the blood pressure or expand the coronary vessel. It should be noted that potassium channels are widely distributed in other smooth muscles such as trachea, intestinal tract, uterus, etc. The activation of the potassium channel is also known to relax these smooth muscles. It follows that a compound which permits to activate these potassium channels is useful as a therapeutic or a prophylactic agent for hypertension, angina pectoris, asthma, etc.

Some compounds which permit activating a potassium channel are known to the art. Specifically, some benzopyran derivatives having guanidino groups at 4-position are disclosed in, for example, Published Unexamined Japanese Patent Application Nos. 2-172984; 2-134357; and 2-42074. Also, some benzopyran derivatives having certain kind of amidine groups at 4-position and pharmaceutically acceptable salts thereof are disclosed in, for example, Published Unexamined Japanese Patent Application No. 2-300182 and EP 0412531.

[Disclosure of the Invention]

As a result of an extensive research on compounds which permit activating a potassium channel, the present inventors have found novel benzopyran derivatives and pharmaceutically acceptable salts thereof, which permits activating a potassium channel and, thus, is useful for prophylaxis or treatment of various diseases referred to above, arriving at the present invention.

According to the present invention, there are provided novel bensopyran derivatives represented by formula [I] given below:

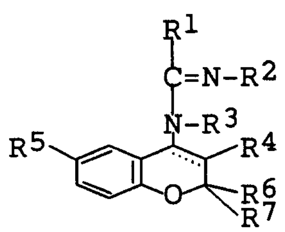

(I)

where

$R^1$ is a lower alkyl group which may have at least one substituent selected from the group consisting of at least one halogen atom, at least one nitro group, at least one lower alkoxycarbonyl group and at least one cyano group; a cycloalkyl group having 3 to 7 carbon atoms; an aryl group or an aralkyl group which may have at least one substituent selected from the group consisting of at least one halogen atom, at least one lower alkyl group, at least one halogen-substituted lower alkyl group, at least one nitro group, at least one lower alkoxycarbonyl group, at least one cyano group, at least one lower alkylsulfonyl group, at least one aminosulfonyl group, at least one acylamino group, at least one lower alkylsulfonylamino group, at least one halogen-substituted acyl group and at least one acyl group; a hetero aryl group which may have at least one substituent selected from the group consisting of at least one halogen atom, at least one lower alkyl group, at least one nitro group, at least one lower alkoxycarbonyl group and at least one cyano group; or a saturated hetero ring group having 3 to 6 carbon atoms and represented by formula

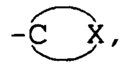

(where X is O, S or N-$R^8$, said $R^8$ denoting a lower alkyl group or an acyl group);

$R^2$ is a hydroxyl group, a lower alkoxy group, a cyano group, a nitro group, a cyanomethyl group or a group represented by formula -A-$R^9$ or

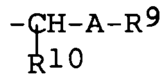

(where A is a carbonyl group or a sulfonyl a group; $R^9$ is a lower alkyl group in which at least one halogen atom may be substituted, a lower alkoxy group, an amino group in which lower alkyl may be substituted; and $R^{10}$ a is hydrogen atom or a lower alkyl group));

$R^3$ is a hydrogen atom or a lower alkyl group;

$R^4$ is a hydrogen atom, a hydroxyl group, a nitroxy group, or an acetoxy group;

$R^5$ is a lower alkyl group, a lower alkoxy group in which at least one halogen atom may be substituted, a cyano group, a nitro group, an acyl group or a halogen atom; and

$R^6$ and $R^7$, which may be the same or different, are lower alkyl groups or collectively form an alkylene group having 4 to 6 carbon atoms.

The symbol $\overline{\cdots}$ in formula [I] denotes a single bond or double bond.

When $R^2$ denotes a cyano group, $R^1$ does not represent an unsubstituted lower alkyl group, unsubstituted aralkyl group, unsubstituted aryl group or a lower alkyl-substituted aryl group.

Some of the terms used in the present specification for defining various compounds should be construed as follows:

First of all, "lower alkyl group" denotes a linear or branched alkyl group having 1 to 5, preferably, 1 to 4 carbon atoms. To be more specific, a lower alkyl group includes, for example, methyl, ethyl, propyl, isopropyl, butyl, isobutyl, sec-butyl, tert-butyl, and pentyl.

"Lower alkoxy group" denotes a linear or branched alkoxy group having 1 to 5, preferably, 1 to 4 carbon atoms. Specifically, a lower alkoxy group includes, for example, methoxy, ethoxy, propoxy, isopropoxy, butoxy, isobutoxy, sec-butoxy, tert-butoxy and pentoxy.

"Lower alkoxycarbonyl group" denotes alkoxycarbonyl group derived from the lower alkoxy group described above and having 1 to 6, preferably, 1 to 4 carbon atoms. Specifically, a lower alkoxycarbonyl group includes, for example, methoxycarbonyl, ethoxycarbonyl, propoxycarbonyl, isopropoxycarbonyl, butoxycarbonyl, isobutoxycarbonyl, sec-butoxycarbonyl, tertbutoxycarbonyl, and pentoxycarbonyl.

"Halogen atom" includes, for example, fluorine, chlorine, bromine and iodine atoms.

"Halogen-substituted lower alkyl group" denotes the lower alkyl group defined previously which has at least one halogen atom defined above substituted for a hydrogen atom. Specifically, a halogen-substituted lower alkyl group includes, for example, trifluoromethyl, trichloromethyl, pentafluoroethyl and pentachloroethyl. Trifluoromethyl is particularly preferred.

"Lower alkylsulfonyl group" denotes a sulfonyl group derived from the lower alkyl group defined previously. Specifically, a lower alkylsulfonyl group includes, for example, methanesulfonyl, ethanesulfonyl, propanesulfonyl, butanesulfonyl, and pentanesulfonyl.

"Lower alkylsulfonylamino group" denotes a sulfonylamino group derived from the lower alkyl group defined previously. Specifically, a lower alkylsulfonylamino group includes, for example, methanesulfonylamino, ethanesulfonylamino, propanesulfonylamino, butanesulfonylamino, and pentanesulfonylamino.

"Cycloalkyl group" denotes a cycloalkyl group having 3 to 7 carbon atoms. Specifically, a cycloalkyl group includes, for example, cyclopropyl, cyclobutyl, cyclopentyl, cyclohexyl, and cycloheptyl.

"Aralkyl group" denotes an arylalkyl group. Specifically, an aralkyl group includes, for example, benzyl, 1-phenylethyl, 2-phenylethyl, 1-phenylpropyl, 2-phenylpropyl and 3-phenylpropyl. Particularly preferable are benzyl, 1-phenylethyl and 2-phenylethyl groups.

"Aryl group" includes, for example, phenyl, biphenyl, naphthyl, anthryl and phenanthryl. Phenyl is preferable.

"Hetero aryl group" includes, for example, 2-pyridinyl, 3-pyridinyl, 4-pyridinyl, pyradinyl, 2-pyrimidinyl, 4-pyrimidinyl, 5-pyrimidinyl, 3-pyridadinyl, 4-pyridadinyl, 2-furanyl, 3-furanyl, 2-thienyl, 3-thienyl, 2-pyrrolyl, 3-pyrrolyl, pyridine-N-oxide-2-yl, pyridine-N-oxide-3-yl, and pyridine-N-oxide-4-yl. Preferably, a hetero aryl group should represent 2-pyridinyl, 3-pyridinyl, 4-pyridinyl, pyradinyl, pyridine-N-oxide-2-yl and pyridine-N-oxide-3-yl groups.

"Acyl group" includes, for example, formyl, acetyl, propionyl, butyryl, isobutyryl and valeryl.

"Acylamino group" denotes an amino group derived from the acyl group referred to above. Specifically, an acylamino group includes, for example, acetylamino, propionylamino, butyrylamino, isobutyrylamino and valerylamino.

"Halogen-substituted acyl group" denotes the acyl group defined above which has at least one halogen atom substituted for a hydrogen atom of the acyl group. Specifically, a halogen-substituted acyl group includes, for example, trifluoroacetyl and trichloroacetyl.

"Saturated hetero ring group having 3 to 6 carbon atoms" denotes a ring having one hetero atom and includes, for example, 2-pyrrolydinyl, 3-pyrrolydinyl, 2-piperidinyl, 3-piperidinyl, 2-tetrahydrofuranyl, 3-tetrahydrofuranyl, 2-tetrahydrothienyl and 3-tetrahydrothienyl.

"Alkylene group having 4 to 6 carbon atoms" includes, for example, tetramethylene-1,4-biyl, pentamethylene-1,5-biyl and hexamethylene-1,6-biyl.

"Pharmaceutically acceptable salt" includes, for example, inorganic acid salts such as hydrogen chloride salt, hydrogen bromide salt, sulfuric acid salt, phosphoric acid salt and nitric acid salt; organic acid salts such as acetic acid salt, propionic acid salt, succiic acid salt, glycolic acid salt, lactic acid salt, malic acid salt, tartaric acid salt, citric acid salt, maleic acid salt, fumaric acid salt, methanesulfonic acid salt, p-toluenesulfonic acid salt, and ascorbic acid salt; and salts with various amino acids such as aspartic acid salt and glutamic acid salt. However, the pharmaceutically acceptable salt is not restricted to those exemplified above.

The compound represented by formula [I] has 0, 1 or plural asymmetric carbon atoms. Where the compound has one asymmetric carbon atom, it is possible for a optically pure isomer, a mixture of enantiomer in optional mixing ratio or a racemic mixture of the enantiomers to be present. Where the compound has plural asymmetric carbon atoms, it is possible for optically pure diastereomer, a racemic mixture thereof, combinations thereof and a mixture of optional mixing ratio to be present. In addition, it is possible for E- and Z-stereoisomers to be present in the amidine portion. Further, it is possible for a resonance structure to be present in the amidine portion. The present invention covers all of these isomers.

The benzopyran derivative of the present invention can be prepared as described in the following. Needless to say, however, the preparing method is not restricted to those exemplified below.

The flow chart of preparing method 1 is as given below:

Preparing method 1

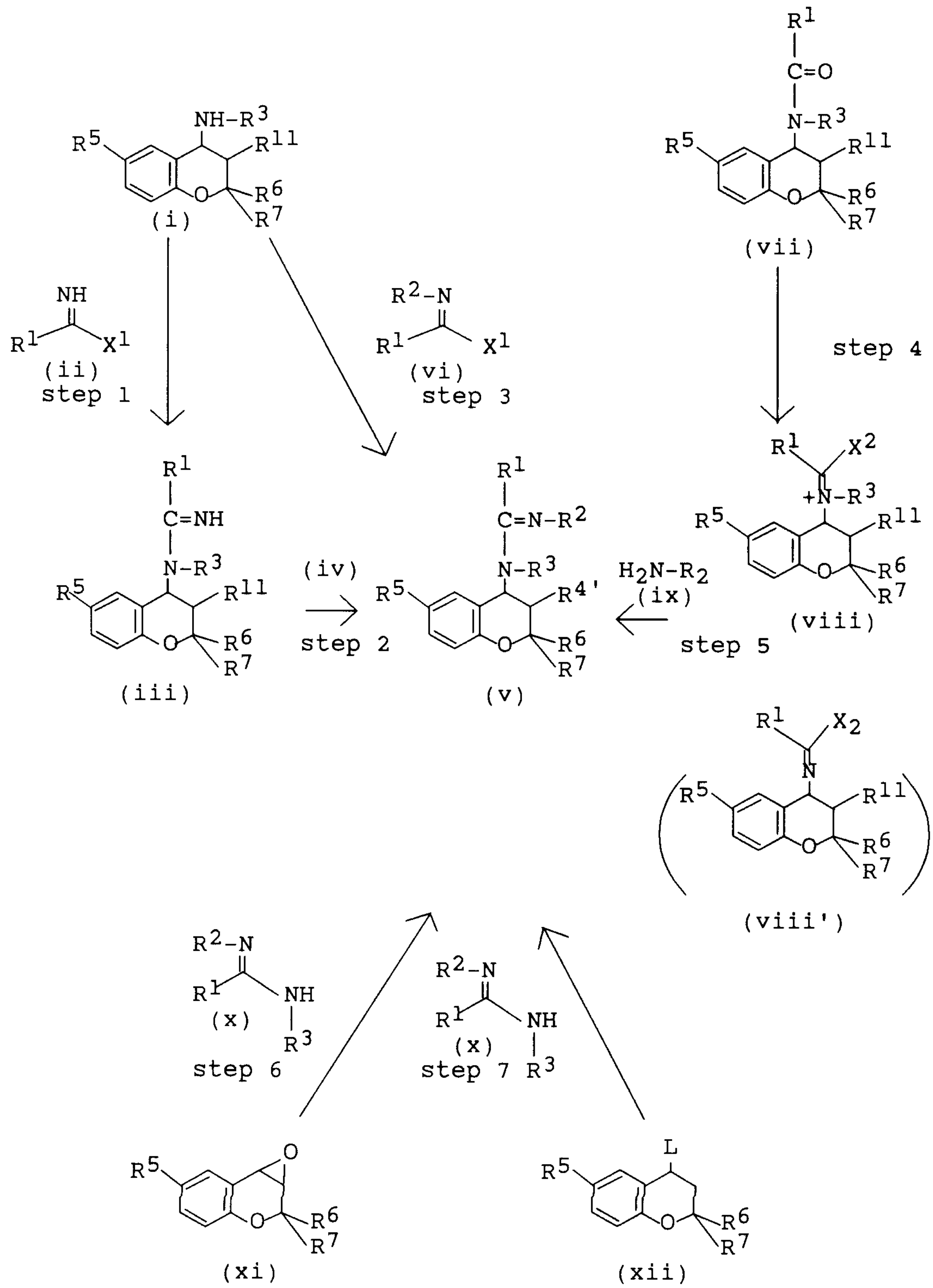


Preparing method 1 covers the synthesis of compound (v), i.e., compound of formula [I] in which ⎓ denotes a single bond, and $R^4$ is a hydrogen atom or a hydroxyl group, $R^1$, $R^2$, $R^3$, $R^5$, $R^6$, $R^7$ in compound (v) being as defined previously.

⟨Step 1⟩

Compound (iii) can be prepared by the reaction between compound (i) and compound (ii) or a salt thereof by the method disclosed in, for example, Ber. 88,1267 (1955). The reaction should be carried out in a solvent which does not inhibit the reaction itself in the presence or absence of a base and under a cooled or warmed condition.

$R^1$, $R^3$, $R^5$, $R^6$, $R^7$ in compound (iii) are as noted above, and $R^{11}$ is H, OH or $OR^{12}$, ($R^{12}$ being a suitable protecting group for a hydroxyl group). The protecting group $R^{12}$ for a hydroxyl group includes, for example, a silyl-type such as trimethylsilyl or t-butyldimethylsilyl; an acyl-type such as acetyl or benzoyl; an alkoxycarbonyl-type such as t-butoxycarbonyl or benzyloxycarbonyl; an alkyl-type such as benzyl or t-butyl; and tetrahydropyranyl group.

Compound (i), in which $R^3$, $R^5$, $R^6$, $R^7$ and $R^{11}$ are as noted above, can be prepared by the method disclosed in, for example, J. Med. Chem. 33,2667 (1990) or Published Unexamined Japanese Patent Application No. 2-134357 or a method similar thereto.

Compound (ii), in which $R^1$ is as noted above and $X^1$ is an alkoxy or alkylthio group, can be readily derived from a corresponding nitrile or amide by known methods described in, for example, Organic Functional Group Preparations 2nd, vol. 3, chap. 8, ACADEMIC PRESS, INC. Compound (ii) includes unstable compounds and, thus, it is desirable in some cases to carry out the reaction under a cooled condition for obtaining a satisfactory result. Where compound (ii) is a salt, a salt with any kind of acid is basically acceptable. However, a hydrogen chloride salt is most generally used in view of the synthetic method of imidate.

The solvent which does not inhibit the reaction itself includes, for example, aprotic solvents such as benzene, toluene, methylene chloride, chloroform, ether, dioxane, tetrahydrofuran, acetonitrile, dimethylformamide and dimethylsulfoxide; alcoholic solvents such as methanol and ethanol; and a mixed solvent thereof.

The base used in reaction 1 includes, for example, pyridine, N-methylmorpholine, triethylamine, sodium hydride, sodium hydroxide, sodium bicarbonate and sodium carbonate.

⟨Step 2⟩

Compound (v) can be prepared by the reaction between compound (iii) obtained in reaction 1 and compound (iv) described in the following. The reaction can be carried out by or according to the known method described in, for example, Tetrahydron, 25,5437 (1969) in a solvent which does not inhibit the reaction itself and in the presence or absence of a base.

Compound (iv) is used as a reagent for introducing $R^2$ into the =NH portion of compound (iii). In the present invention, compounds generally known as reagents for introducing $R^2$ into an amino group ($-NH^2$) can be used as compound (iv). Specific examples of compound (iv) include acylation agents such as acetyl chloride, acetic anhydride, propionyl chloride, trichloroacetyl chloride, trichloroacetic anhydride, trifluoroacetyl chloride, and trifluoroacetic anhydride; sulfonylation agents such as methanesulfonyl chloride, methanesulfonic anhydride, trifluoromethanesulfonyl chloride, and trifluoromethanesulfonic anhydride; alkoxycarbonylation agents such as methyl chloroformate, ethyl chloroformate, isopropyl chloroformate and t-butyl chloroformate; halogenated cyanogens such as cyanogen chloride, and cyanogen bromide; isocyanates or isothiocyanates such as methyl isocyanate, ethyl isocyanate, methyl isothiocyanate, and ethyl isothiocyanate; nitrating agents such as acetylnitrate; and other compounds such as methyl chloroacetate and ethyl chloropropionate.

In the case of using cyanogen bromide as compound (iv), compound (v) in which $R^2$ is CN can be obtained by the reaction described above. In the case of using a chloroformic acid ester, e.g., ethyl chloroformate, as compound (iv), obtained is compound (v) in which $R^2$ is $CO_2Et$. In the case of using an isocyanate, e.g., methyl isocyanate, as compound (iv), obtained is compound (v) in which $R^2$ is CONHMe. Further, in the case of using $\alpha$-halocarboxylic acid ester, e.g., ethyl $\alpha$-bromopropionate, as compound (iv), obtained is compound (v) in which $R^2$ is $CHMe\text{-}CO_2Et$.

⟨Step 3⟩

Step 1 and 2 described above can be replaced by step 3 to obtain compound (v). In the step 3, reaction is carried out between compound (i) and compound (vi), in which $R^1$, $R^2$ and $X^1$ are the same as defined previously, according to the method disclosed in, for example, Synthesis, 1978,673; Synthesis, 1980,213; and J. Org. Chem., 26,412 (1961).

The above reaction can be carried out in a suitable solvent or without using any solvent in the presence or absence of a base, under a cooled or warmed condition. The solvents used in this reaction include, for example, aprotic solvents such as benzene, toluene, methylene chloride, chloroform, ether, dioxane, tetrahydrofuran, acetonitrile, dimethylformamide and dimethylsulfoxide; protic solvents such as water, methanol and ethanol; and a mixture thereof.

〈Step 4〉

Compound (viii) or (viii') can be prepared from compound (vii) by the method disclosed in, for example, Org. Synth., 26,97 (1946); J. Chem. Soc., 1959,2865; and J. Org. Chem., 30,2381 (1965).

$R^1$, $R^3$, $R^5$, $R^6$, $R^7$ and $R^{11}$ in compound (viii) are as defined previously, and $X^2$ is a halogen atom such as chlorine or bromine, a alkoxy or a alkylthio group. Compound (viii') is a specific form of compound (viii) in which $R^3$ is a hydrogen atom.

$R^1$, $R^3$, $R^5$, $R^6$, $R^7$ and $R^{11}$ in compound (vii) are as defined previously. Compound (vii) can be prepared by the method disclosed in, for example, J. Med. Chem., 33, 2667 (1990).

According to the method described in the literature noted above, compound (vii) is reacted with, for example, phosphorus pentachloride, phosphorus oxychloride, thionyl chloride or phosgene to form compound (viii) or (viii') in which $X^2$ is a halogen atom. Also, compound (viii) or (viii') in which $X^2$ is an alkoxy group can be obtained by the reaction between compound (vii) and triethyloxonium tetrafluoroborate. Further, compound (viii) or (viii') in which $X^2$ is an alkylthio group can be obtained by a two-step reaction. In the reaction, compound (vii) is converted into a thioamide derivative by the reaction with diphosphorus pentasulfide or Lawesson's reagent, followed by carrying out a reaction between the resultant thioamide derivative and an alkylating agent in the presence of a base such as sodium hydride so as to obtain compound (viii) or (viii') in which $X^2$ is an alkylthio group.

〈Step 5〉

Compound (v) can also be prepared by the reaction between compound (viii) or (viii') and compound (ix), in which $R^2$ is as defined previously, by the method disclosed in, for example, J. Med. Chem., 23,690 (1980) and Aust. J. Chem., 29,357 (1976).

The above mentioned reaction can be carried out in a suita ble solvent or without using a solvent in the presence or absence of a base, under a cooled or warmed condition. The solvents used in this reaction include, for example, aprotic solvents such as benzene, toluene, methylene chloride, chloroform, ether, dioxane, tetrahydrofuran, acetonitrile, dimethylformamide and dimethylsulfoxide; protic solvents such as water, methanol and ethanol; and a mixture thereof. Also, the bases used in this reaction include, for example, pyridine, N-methyl-morpholine, triethylamine, sodium hydride, sodium hydroxide, sodium bicarbonate, sodium carbonate.

〈Step 6〉

Compound (v) can also be prepared by the reaction between compound (xi) and compound (x) in a suitable solvent or without using a solvent in the presence of a Lewis acid or a base or in the absence of an acid and a base, under a cooled or warmed condition.

$R^5$, $R^6$, and $R^7$ in compound (xi) are as defined previously. Compound (xi) can be prepared by the method disclosed in, for example, J. Med. Chem., 26,1582 (1983). On the other hand, $R^1$, $R^2$, and $R^3$ in compound (x) are as defined previously.

Suitable solvents used in this reaction include, for example, aprotic solvents such as benzene, toluene, methylene chloride, chloroform, ether, dioxane, tetrahydrofuran, acetonitrile, dimethyl formamide and dimethylsulfoxide; protic solvents such as water, methanol and ethanol; and a mixture thereof.

The Lewis acids used in this reaction include, for example, boron trifluoride ether complex, zinc chloride, aluminum chloride and titanium tetrachloride.

The bases used in this reaction include, for example, pyridine, N-methylmorpholine, triethylamine, sodium hydride, sodium hydroxide, sodium bicarbonate, and sodium carbonate.

A particularly satisfactory result can be obtained if the reaction mentioned above is carried out under a cooled condition in the presence of a Lewis acid.

⟨Step 7⟩

Further, compound (v) can also be prepared by the reaction between compound (xii) and compound (x) noted above in a suitable solvent or without using a solvent in the presence or absence of a base, under a cooled or warmed condition.

$R^5$, $R^6$, and $R^7$ in compound (xii) are as defined previously. On the other hand, L in compound (xii) represents a leaving group such as chlorine, bromine, p-toluenesulfonyloxy, methanesulfonyloxy, trifluoroacetoxy or trifluoromethanesulfonyloxy group. Compound (xii) can be prepared by the method disclosed in, for example, J. Med. Chem., 33,492 (1990) or Published Unexamined Japanese Patent Application No. 2-134357 or a method similar thereto.

Suitable solvents used in this reaction include, for example, aprotic solvents such as benzene, toluene, methylene chloride, chloroform, ether, dioxane, tetrahydrofuran, acetonitrile, dimethyl formamide and dimethylsulfoxide; protic solvents such as water, methanol and ethanol; and a mixture thereof.

The bases used in this reaction include, for example, pyridine, N-methylmorpholine, triethylamine, sodium hydride, sodium hydroxide, sodium bicarbonate, and sodium carbonate.

A good result can be obtained in some cases, if a phase-transfer catalyst is used in the reaction mentioned above.

Among the various compounds used in preparing method 1 described above, some of compounds (ii), (iv) and (ix) are commercially available. Also, compound (ii) can be prepared easily from the corresponding nitrile by Pinner's method or a base catalyzed method. Compound (ii) can also be prepared easily from the corresponding amide by the reaction in step 4. Compound (vi) can be prepared from compound (ii) by a method substantially equal to that in step 2. Further, compound (x) can be prepared easily by the reaction between compound (vi) and an amine by a method substantially equal to that in step 3.

In each step, the hydroxyl group in the 3-position of the benzopyrane ring may desirably be protected appropriately during the reaction, followed by removing the protective group in a suitable stage after the reaction. Alternatively, the reaction can be carried out without protecting the hydroxyl group noted above. Further, it is possible for all the compounds in each of the steps to be in the form of salts such as hydrogen chloride salt, hydrogen bromide salt, sulfuric acid salt, sulfonic acid salt, acetic acid salt and trifluoroacetic acid salt.

The flow chart of preparing method 2 is as shown below:

Preparing method 2

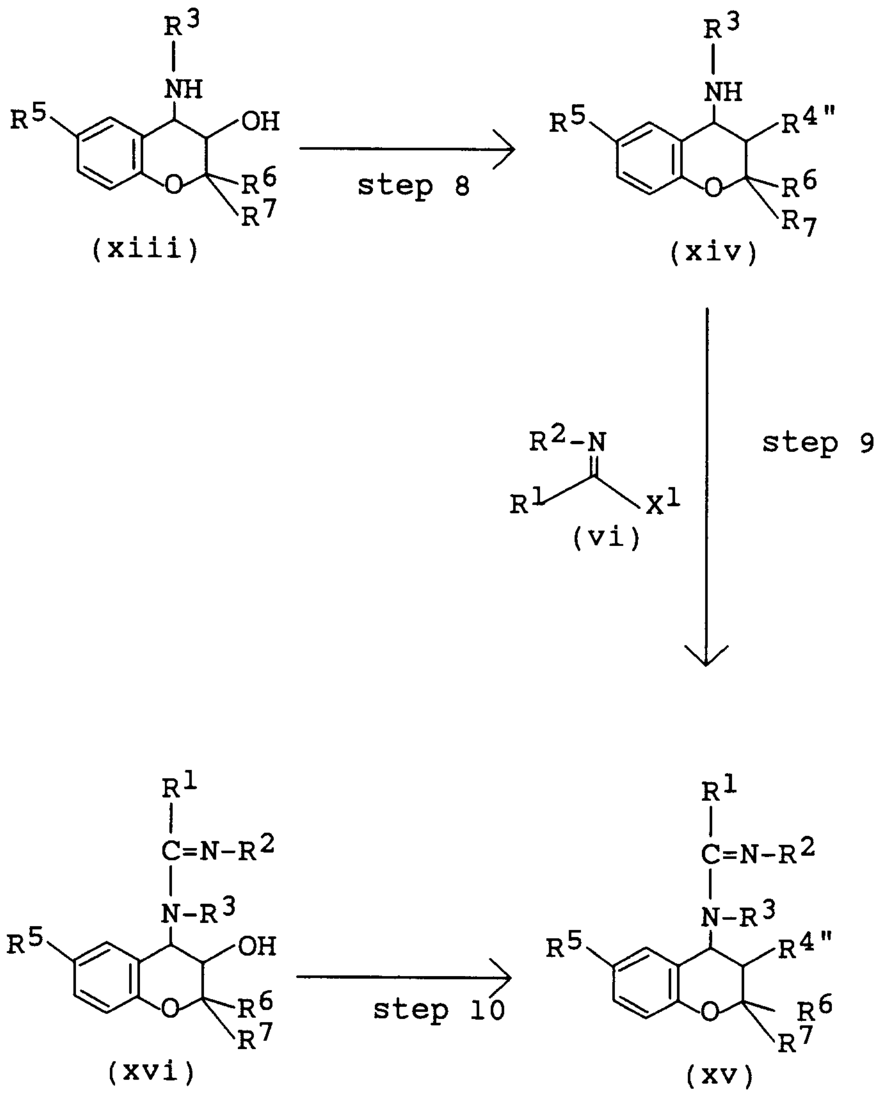


(xiii) (xiv) (vi) (xvi) (xv)

Preparing method 2 is directed to the synthesis of compound (xv) belonging to formula [I], in which $\underline{\cdots}$ denotes a single bond and $R^4$ is a nitroxy group or an acetoxy group. $R^1$, $R^2$, $R^3$, $R^5$, $R^6$ and $R^7$ in compound (xv) are as defined previously, and $R^{4}$" is a nitroxy or acetoxy group.

⟨Step 8⟩

Compound (xiv) can be prepared by converting compound (xiii) into a nitrate ester in a solvent which does not inhibit the nitrating reaction itself under a cooled condition by the ordinary chemical technique, or by acetylating compound (xiii) by the ordinary method. $R^3$, $R^{4}$", $R^5$, $R^6$ and $R^7$ in each of compounds (xiv) and (xiii) are as defined previously.

Compound (xiii) can be prepared by the method disclosed in, for example, J. Med. Chem., 33,2667 (1990) or a method substantially equal to said method.

The chemical technique for conversion of compound (xiii) into a nitrate ester includes, for example, the reaction with acetyl nitrate or the reaction with nitronium tetrafluoroborate in acetonitrile.

The acetylating technique includes, for example, the reaction with acetic anhydride or the reaction with acetyl chloride in pyridine.

In order to prevent the reaction at the amino group at 4-position, it is desirable in some cases to protect the amino group in advance with, for example, t-butoxycarbonyl or benzyloxycarbonyl group. In this case, the protection group should be removed after conversion of the hydroxyl group into $R^{4}$". Unless the

particular treatment is performed, a desired reaction fails to proceed satisfactorily in some cases.

⟨Step 9⟩

Compound (xv) can be prepared by the reaction between compound (xiv) and compound (vi) under the conditions substantially equal to those in step 3 included in preparing method 1.

⟨Step 10⟩

Compound (xv) can be prepared from compound (xvi), in which $R^1$, $R^2$, $R^3$, $R^5$, $R^6$ and $R^7$ are as defined previously, under the reacting conditions substantially equal to those in step 8.

The flow chart for preparing method 3 is as follows:

Preparing method 3

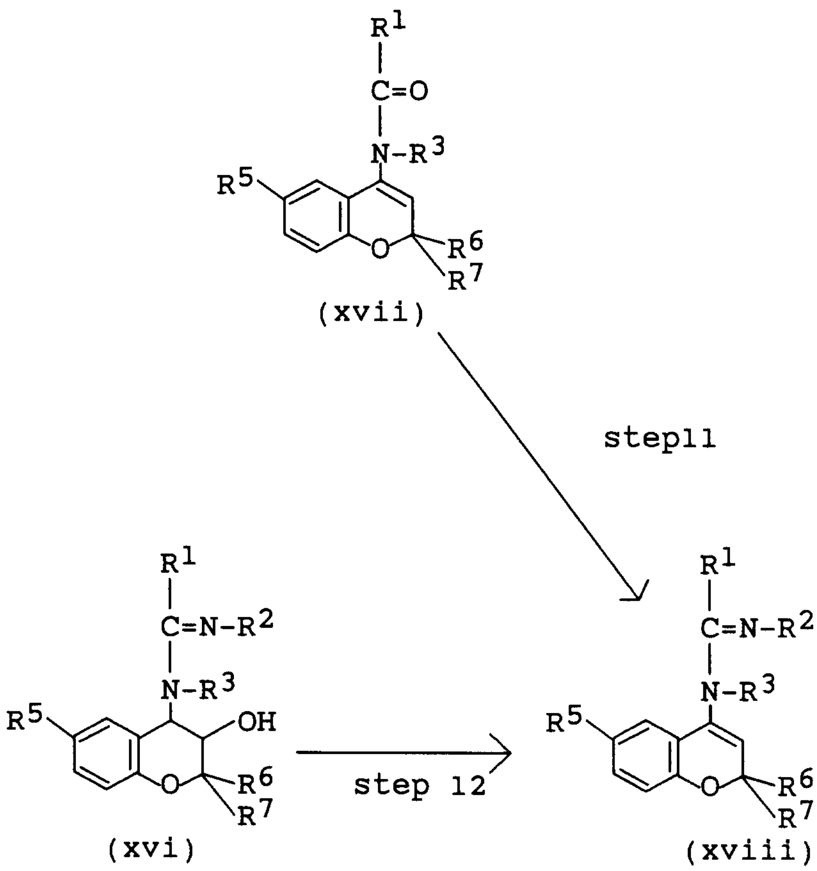


(xvii)

(xvi)

(xviii)

Preparing method 3 is directed to the synthesis of compound (xviii) belonging to formula [I], in which ⎓ denotes a double bond and $R^4$ is a hydrogen atom. $R^1$, $R^2$, $R^3$, $R^5$, $R^6$ and $R^7$ in compound (xviii) are as defined previously.

⟨Step 11⟩

Compound (xviii) can be prepared from compound (xvii), in which $R^1$, $R^3$, $R^5$, $R^6$ and $R^7$ are as defined previously. On the other hand, compound (xvii) can be prepared by the method disclosed in, for example, J. Med. Chem., 33,2667 (1990) or a similar method. The conversion from compound (xvii) into compound (xviii) is basically equal to that from compound (vii) into compound (v) included in preparing method 1, i.e., steps 4 and 5. Thus, the techniques described previously can be employed for this conversion.

⟨Step 12⟩

Compound (xviii) can also be synthesized from compound (xvi) in accordance with the method disclosed in J. Med. Chem., 33,2667 (1990) or by the ordinary dehydration method using a Dean-Stark apparatus. Alternatively, compound (xviii) can be synthesized by activating the hydroxyl group in 3-position of compound (xvi) with, for example, methanesulfonyl or trifluoromethanesulfonyl, followed by removing the particular hydroxyl group under a basic condition.

Where the compound of the present invention and a pharmaceutically acceptable salt thereof is used as a medicine, the compound or a salt thereof is mixed in general with a pharmaceutically acceptable additives such as a carrier, an excepient, a diluent and a solubilizer. The resultant composition can be administered orally or non-orally with safety in the form of tablets including a sugar-coated tablet and film-coated tablet, capsules, powder, granules, injection solution, dripinfusion solution, suppository or cataplasm.

The amount of administration to a patient, which depends on the sex, age, weight or symptom of the disease of the patient, should fall within a range of between about 1 mg and 500 mg per day for an adult in the case of the oral administration. The medicinal composition should be administered once a day or several times a day in the amount noted above. Of course, the amount and manner of administration need not be restricted to those described above.

The compound of the present invention and a pharmaceutically acceptable salt thereof permit activating the potassium channel so as to produce a continuous action of lowering the blood pressure and, an action of relaxing capillary vessels, thus, these are useful as a therapeutic agent for hypertension.

The particular compound and the salt thereof also exhibit an action of selectively increasing the coronary blood flow and, thus, are useful as a therapeutic agent for diseases in the cardiovascular system such as angina pectoris and cardiac failure.

Further, the particular compound and the salt thereof produce an action of relaxing smooth muscles other than the blood vessel smooth muscles described above and, thus, are useful as a therapeutic agent for tumor in the digestive organ, irritable colon syndrome, diverticulosis, reversible trachea closure, asthma, premature birth, incontinence of urine, and cerebrovascular disease.

[Best Mode of Embodying the Invention]

Let us describe some Examples of the present invention. Of course, the present invention is not restricted to these Examples alone. Also, the abbreviations used in the Examples are defined as follows:

THF : tetrahydrofuran
DMF : dimethylformamide
DMSO: dimethylsulfoxide
NMR : nuclear magnetic resonance spectrum
ppm : parts per million

Example 1

i) Added to 1 mℓ of dry dimethylformamide were 1.0g of trans-4-(acetamido)-6-cyano-3,4-dihydro-2,2-dimethyl-2H-1-benzopyran-3-ol, which is described in J. Med. Chem., 33,2667 (1990), 1.74g of t-butyldimethylchlorosilane, and 1.74g of imidazole. The resultant mixture was kept stirred at 40°C for 4 days under a nitrogen gas atmosphere. Then, a sodium bicarbonate solution was added to the reaction solution, followed by extraction with ethyl acetate and, then, washing with saline. The washed organic layer was dried and condensed, followed by adding hexane to the resultant residue. After stirring, the mixture was filtered to give 1.35g of trans-4-(acetamido)-3-(t-butyldimethylsilyloxy)-6-cyano-3,4-dihydro-2,2-dimethyl-2H-1-benzopyran.

$^1$H NMR (CDCℓ$_3$/ppm) δ 0.12(s, 3H), 0.13(s, 3H), 0.90(s, 9H), 1.27(s, 3H), 1.44(s, 3H), 2.12(s, 3H), 3.68(d, J=9Hz, 1H), 5.10(dd, J=9Hz, 9Hz, 1H), 5.55(d, J=9Hz, 1H), 6.83(d, J=8.5Hz, 1H), 7.41(1H), 7.46(1H).

ii) Added to 15 mℓ of dry pyridine were 1.5g of trans-4-(acetamido)-3-(t-butyldimethylsilyloxy)-6-cyano-3,4-dihydro-2,2-dimethyl-2H-1-benzopyran and 3.6g of phosphorus pentasulfide. The resultant mixture was kept stirred at 80°C for 20 hours under a nitrogen gas atmosphere. Then, a sodium bicarbonate solution and saline were added to the reaction solution, followed by extraction with ethyl acetate. The organic layer was dried over magnesium sulfate and was concentrated, followed by subjecting to a silica gel column chromatography (chloroform : methanol = 50 : 1) to give 1.52g of trans-3-(t-butyldimethylsilyloxy)-6-cyano-3,4-dihydro-2,2-dimethyl-4-(thioacetamido)-2H-1-benzopyran.

$^1$H NMR (CDC$\ell_3$/ppm) δ 0.11(s, 3H), 0.13(s, 3H), 0.90(s, 9H), 1.32(s, 3H), 1.46(s, 3H), 2.66(s, 3H), 3.85(d, J=9Hz, 1H), 5.99(br t, 1H), 6.85(d, J=8.5Hz, 1H), 7.24(br d, 1H), 7.41(1H), 7.52(1H).

iii) 537 mg of trans-3-(t-butyldimethylsilyloxy)-6-cyano-3,4-dihydro-2,2-dimethyl-4-(thioacetamido)-2H-1-benzopyran was dissolved in 5 mℓ of dry DMF, followed by adding 77 mg of a 60% sodium hydride to the resultant solution which was kept stirred. When a hydrogen gas generation was ceased, 0.104 mℓ of methyl iodide was added to the solution, which was kept stirred for 20 hours at room temperature. The reaction solution was evaporated under a reduced pressure, followed by adding saline to the residue and, then, extracting twice with ethyl acetate. The organic layer was dried over magnesium sulfate, followed by evaporating the solvent under a reduced pressure to give 1g of methyl N-[trans-3-(t-butyldimethylsilyloxy)-6-cyano-3,4-dihydro-2,2-dimethyl-2H-1-benzopyran-4-yl]thioacetimidate.

$^1$H NMR (CDC$\ell_3$/ppm) δ 0.00(s, 3H), 0.08(s, 3H), 0.88(s, 9H), 1.28(s, 3H), 1.46(s, 3H), 2.20(s, 3H), 2.28(s, 3H), 3.91(d, J=8.5Hz, 1H), 4.61(d, J=8.5Hz, 1H), 6.84(d, J=8.5Hz, 1H), 7.01(1H), 7.40(1H).

iv) 300mg of methyl N-[trans-3-(t-butyldimethylsilyloxy)-6-cyano-3,4-dihydro-2,2-dimethyl-2H-1-benzopyran-4-yl]thioacetimidate and 155 mg of hydroxylamine hydrochloride were dissolved in 1.5 mℓ of dry pyridine, followed by stirring the resultant solution at room temperature for 7 hours under a nitrogen gas atmosphere. Then, saline was added to the reaction solution, followed by extraction with chloroform. After drying and concentration, the resultant residue was subjected to a silica gel column chromatography (chloroform: methanol = 50 : 1) to give 148 mg of N-[trans-3-(t-butyldimethylsilyloxy)-6-cyano-3,4-dihydro-2,2-dimethyl-2H-1-benzopyran-4-yl]-N'-hydroxyacetamidine.

$^1$H NMR (CDC$\ell_3$/ppm) δ 0.11(s, 3H), 0.13(s, 3H), 0.93(s, 9H), 1.26(s, 3H), 1.47(s, 3H), 2.00(s, 3H), 3.61(d, J=9Hz, 1H), 4.33(dd, J=9Hz, 11Hz, 1H), 5.28(d, J=11Hz, 1H), 6.84(d, J=8.5Hz, 1H), 7.44(1H), 7.53(1H).

v) 119 mg of N-[trans-3-(t-butyldimethylsilyloxy)-6-cyano-3,4-dihydro-2,2-dimethyl-2H-1-benzopyran-4-yl]-N'-hydroxyacetamidine was dissolved in 5 mℓ of THF, followed by adding 1 mℓ of tetrabutylammonium fluoride (1M solution/THF) to the resultant solution. The resultant mixture was stirred for 2.5 hours at room temperature, followed by removing the solvent by evaporation. Saline was added to the residue, followed by extraction with chloroform. The organic layer was dried over magnesium sulfate and concentrated, followed by subjecting the resultant residue to a silica gel column chromatography (ethyl acetate). The product thus obtained was recrystallized from chloroform-ether to give 40 mg of N-(trans-6-cyano-3,4-dihydro-2,2-dimethyl-3-hydroxy-2H-1-benzopyran-4-yl)-N'-hydroxyacetamidine.

Melting point: 154 - 156°C

$^1$H NMR (CDC$\ell_3$-$CD_3OD$/ppm) δ 1.28(s, 3H), 1.52(s, 3H), 2.06(s, 3H), 4.33(d, J=9.3Hz, 1H), 6.86(d, J=8.5Hz, 1H), 7.46(d, J=8.5Hz, 1H), 7.65(1H).

## Example 2

i) 0.5 mℓ of dry pyridine was added under a nitrogen gas atmosphere to a mixture consisting of 100 mg of crude methyl N-[trans-3-(t-butyldimethylsilyloxy)-6-cyano-3,4-dihydro-2,2-dimethyl-2H-1-benzopyran-4-yl]thioacetimidate obtained in Example 1, step iii) and 62 mg of O-methylhydroxyamine hydrochloride. The mixture was kept stirred overnight. Then, saline was added to the mixture, followed by extraction with chloroform. The organic layer was dried and concentrated and, then, subjected to a thin-layer chromatography (chloroform : methanol = 50 : 1) to give 45.4 mg of N-[trans-3-(t-butyldimethylsilyloxy)-6-cyano-3,4-dihydro-2,2-dimethyl-2H-1-benzopyran-4-yl]-N'-methoxyacetamidine.

$^1$H NMR (CDC$\ell_3$/ppm) δ 0.09(s, 3H), 0.13(s, 3H), 0.93(s, 9H), 1.26(s, 3H), 1.46(s, 3H), 1.99(s, 3H), 3.58(d, J=9Hz, 1H), 4.31(dd, J=9Hz, 11Hz, 1H), 5.22(d, J=11Hz, 1H), 6.83(d, J=8.5Hz, 1H), 7.44(dd, J=2Hz, 8.5Hz, 1H), 7.53(d, J=2Hz, 1H).

ii) 400 mg of N-[trans-3-(t-butyldimethylsilyloxy)-6-cyano-3,4-dihydro-2,2-dimethyl-2H-1-benzopyran-4-yl]-N'-methoxyacetamidine was dissolved in 3 mℓ of THF, followed by adding 1.5 mℓ of tetrabutylammonium fluoride (1M solution/THF) to the resultant solution. The resultant mixture was kept stirred for 3.5 hours, followed by evaporating the solvent under a reduced pressure. The residue thus obtained was subjected to a silica gel chromatography (ethyl acetate : methanol = 99.5 : 0.5), followed by recrystallization from ethyl acetate-hexane to give 254 mg of N-(trans-6-cyano-3,4-dihydro-2,2-dimethyl-3-hydroxy-2H-1-benzopyran-4-yl)-N'-methoxyacetamidine in the form of an isomer mixture.

Melting point: 163.2 - 164.5°C

$^1$H NMR (acetone-$d_6$/ppm) δ 1.25 and 1.27(s,s, 3H), 1.44 and 1.49(s,s, 3H), 1.99 and 2.00(s,s, 3H), 3.61 and 3.62(s,s, 3H), 3.71(dd), 4.63 and 4.82(t, 1H), 5.22 and 5.45(d,d, 1H), 5.5 and 5.63(br d, br d, 1H), 6.89(d, 1H), 7.53(1H), 7.63 and 7.81(1H).

Example 3

i) Dissolved in 10 mℓ of dry DMF were 1.76g of trans-6-cyano-3,4-dihydro-2,2-dimethyl-4-(3-pyridinecarboxamido)-2H-1-benzopyran-3-ol, which is disclosed in Published Unexamined Japanese Patent Application No. 59-219278, 0.98g of t-butyldimethylchlorosilane and 0.84g of imidazole. The resultant solution was stirred at room temperature. Then, 0.98g of t-butyldimethylchlorosilane and 0.84g of imidazole were added 1, 2 and 3 days later, respectively. The solvent was removed by evaporation under a reduced pressure 10 days later, followed by adding ethyl acetate to the residue and, then, washing with sodium bicarbonate solution and saline. The extracts were dried and evaporated and, then, subjected to a silica gel column chromatography to give 1.89g of trans-3-(t-butyldimethylsilyloxy)-6-cyano-3,4-dihydro-2,2-dimethyl-4-(3-pyridinecarboxamido)-2H-1-benzopyran.

$^1$H NMR ($CDCℓ_3$-acetone-$d_6$/ppm) δ -0.13(s, 3H), -0.01(s, 3H), 0.75(s, 9H), 1.21(s, 3H), 1.37(s, 3H), 3.83(d, 1H), 5.25(t, 1H), 6.76(d, 1H), 7.2-7.4(m, 3H), 8.16(d, 1H), 8.63(d, 1H), 8.98(s, 1H).

ii) 10g of phosphorus pentasulfide was added to 40 mℓ of dry pyridine solution containing 5.01g of trans-3-(t-butyldimethylsilyloxy)-6-cyano-3,4-dihydro-2,2-dimethyl-4-(3-pyridinecarboxamido)-2H-1-benzopyran while stirring the solution. The resultant mixture was kept stirred at 80°C for 8 days, followed by ice-cooling. Then, a sodium bicarbonate solution was added to the reaction solution, followed by extraction with ethyl acetate and, then, washing with saline. The ethyl acetate layer was dried and concentrated, followed by subjecting the residue to a silica gel column chromatography (ethyl acetate : hexane = 1 : 1) to give 4.74g of trans-3-(t-butyldimethylsilyloxy)-6-cyano-3,4-dihydro-2,2-dimethyl-4-(3-pyridinecarbothioamido)-2H-1-benzopyran.

$^1$H NMR ($CDCℓ_3$/ppm) δ -0.06(s, 3H), 0.09(s, 3H), 0.86(s, 9H), 1.38(s, 3H), 1.49(s, 3H), 4.1(1H), 6.2-(1H), 6.92(d, J=8.5Hz, 1H), 7.35(1H), 7.45(1H), 8.21(1H), 8.4(br d, 1H), 8.46(1H), 8.78(1H).

iii) 701 mg of trans-3-(t-butyldimethylsilyloxy)-6-cyano-3,4-dihydro-2,2-dimethyl-4-(3-pyridinecarbothioamido)-2H-1-benzopyran was dissolved in 7 mℓ of dry DMF, followed by adding 68 mg of sodium hydride to the resultant solution under ice-cooling. The resultant mixture was kept stirred for 20 minutes, followed by adding 0.11 ml of methyl iodide. The mixture was further kept stirred for 6 hours at room temperature, followed by evaporating the solvent under a reduced pressure. Then, ice water was added to the residue, followed by extraction with ethyl acetate. The organic layer was washed with a saturated saline. After drying, the washed organic layer was subjected to a silica gel column chromatography (ethyl acetate : hexane = 3 : 7) to give 627g of methyl N-[trans-3-(t-butyldimethylsilyloxy)-6-cyano-3,4-dihydro-2,2-dimethyl-2H-1-benzopyran-4-yl]-3-pyridinethiocarboximidate in the form of an isomer mixture.

$^1$H NMR ($CDCℓ_3$/ppm) δ -0.14, 0.06, 0.13 and 0.17(s,s,s,s, 6H), 0.91 and 0.98(s,s, 9H), 2.29 and 2.48-(s,s, 3H), 4.07 and 4.10(d, J=8.6Hz, d, J=8.6Hz, 1H), 4.63 and 5.25(d, J=8.6Hz, d, J=8.6Hz, 1H), 6.82 and 6.90(d, J=8.5Hz, d, J=8.5Hz, 1H), 7.00 and 7.19(1H), 7.3-7.5(2H), 7.88 and 8.0(d,d, 1H), 8.7(1H), 8.81 and 8.95(1H).

iv) 627 mg of methyl N-[trans-3-(t-butyldimethylSilyloxy)-6-cyano-3,4-dihydro-2,2-dimethyl-2H-1-benzopyran-4-yl]-3-pyridinethiocarboximidate was dissolved in 3 mℓ of dry diglyme, followed by adding 290 mg of cyanamide. The resultant mixture was stirred at 80°C. Further, cyanamide was added to the mixture in an amount of 270 mg 4 hours later, 550 mg 6 hours later, 450 mg 23 hours later, and 490 mg 31 hours later. Then, the mixture was cooled 46 hours later. The reaction solution was diluted with ethyl acetate, washed with saline, dried over magnesium sulfate and concentrated and, then, subjected to a silica gel chromatography (chloroform : methanol = 50 : 1) for a simplified purification to give N-[trans-3-(t-butyldimethylsilyloxy)-6-cyano-3,4-dihydro-2,2-dimethyl-2H-1-benzopyran-4yl]-N'-cyano-3-pyridinecarboxamidine.

$^1$H NMR ($CDCℓ_3$/ppm) δ -0.02(s, 6H), 0.11(s, 3H), 0.88(s, 9H), 1.34(s 3H), 1.45(s, 3H), 3.97(d, J=7.4Hz, 1H), 5.31(t, 1H), 6.92(d, J=9.0Hz, 1H), 7.4-7.6(3H), 8.1(d, 1H), 8.6-8.8(2H).

v) The entire amount of N-[trans-3-(t-butyldimethylsilyloxy)-6-cyano-3,4-dihydro-2,2-dimethyl-2H-1-benzopyran-4-yl]-N'-cyano-3-pyridinecarboxamidine obtained in step iv) described above was dissolved in 3 mℓ of THF, followed by adding 7.5 mℓ of tetrabutylammonium fluoride (1M solution/THF) to the resultant solution. The resultant mixture was kept stirred for 4 days at room temperature. Then, the reaction solution was concentrated, diluted with ethyl acetate and, then, washed with a saturated saline water. The washed reaction solution was dried and evaporated and, then, subjected to a silica gel column chromatography (ethyl acetate : methanol = 93 : 7), followed by recrystallization from ethyl acetate-hexane to give 329 mg of N'-cyano-N-(trans-6-cyano-3,4-dihydro-2,2-dimethyl-3-hydroxy-2H-1benzopyran-4-yl]-3-pyridinecarboxamidine.

Melting point: 245.2 - 246.3°C

$^1$H NMR (acetone-$d_6$/ppm) δ 1.36(s, 3H), 1.53(s, 3H), 4.01(dd, J=5.6Hz, 9.4Hz, 1H), 5.37(d, J=5.6Hz,

1H), 5.48(br t, 1H), 6.97(d, J=8.5Hz, 1H), 7.6(2H), 7.87(1H), 8.26(1H), 8.61(br d, 1H), 8.79(1H), 9.02(1H).

Example 4

452 mg of methyl N-[trans-3-(t-butyldimethylSilyloxy)-6-cyano-3,4-dihydro-2,2-dimethyl-2H-1-benzopyran-4-yl]-3-pyridinethiocarboximidate obtained in Example 3, step iii) and 214 mg of O-methylhydroxylamine hydrochloride were dissolved in 1.8 mℓ of dry pyridine. The solution was stirred at room temperature for 18 hours, followed by adding ethyl acetate and, then, washing with saline. The organic layer was dried and condensed and, then, subjected to a silica gel chromatography to give 186 mg of N-[trans-3-(t-butyldimethylsilyloxy)-6-cyano-3,4-dihydro-2,2-dimethyl-2H-1-benzopyran-4-yl]-N'-methoxy-3-pyridinecarboxamidine.

$^1$H NMR ($CDCℓ_3$/ppm) δ 0.71(s, 3H), 0.19(s, 3H), 0.99(s, 9H), 1.10(s, 3H), 1.46(s, 3H), 3.69(d, J=10Hz, 1H), 3.91(s, 3H), 4.40(dd, J=10Hz, 10Hz, 1H), 5.40(d, J=10Hz, 1H), 6,78(d, J=8.5Hz, 1H), 7.33(1H), 7.41-(1H), 7.45(1H), 8.03(1H), 8.66(1H), 8.95(1H).

ii) 164 mg of N-[trans-3-(t-butyldimethylsilyloxy)-6-cyano-3,4-dihydro-2,2-dimethyl-2H-1-benzopyran-4-yl]-N'-methoxy-3-pyridinecarboxamidine was dissolved in 1.5 mℓ of THF, followed by adding 0.5 mℓ of tetrabutylammonium fluoride (1M solution/THF) to the resultant solution. The resultant mixture was stirred at room temperature for 24 hours, followed by concentrating the reaction solution under a reduced pressure. Then, ethyl acetate was added to the residue, followed by washing with saline. After drying and concentration, the reaction product was purified with a thin-layer chromatography (chloroform : methanol = 15 : 1), followed by recrystallization from ethyl acetate to give 99 mg of N-(trans-6-cyano-3,4-dihydro-2,2-dimethyl-3-hydroxy-2H-1-benzopyran-4-yl]-N'-methoxy-3-pyridinecarboxamidine.

Melting point: 106.3 - 108.6 °C

$^1$H NMR ($CDCℓ_3$/ppm) δ 1.04(s, 3H), 1.47(s, 3H), 3.4(br, 1H), 3.63(d, J=10Hz, 1H), 3.93(s, 3H), 4.21-(dd, J=10Hz, 11Hz, 1H), 5.36(d, J=11Hz, 1H), 6.83(d, J=8.4Hz, 1H), 7.39(1H), 7.46(1H), 7.71(1H), 7.79-(1H), 8.67(1H), 8.91(1H).

Example 5

i) 510 mg of methyl N-[trans-3-(t-butyldimethylsilyloxy)-6-cyano-3,4-dihydro-2,2-dimethyl-2H-1-benzopyran-4-yl]-3-pyridinethiocarboximidate obtained in Example 3, step iii) and 225 mg of hydroxylamine hydrochloride were treated as in Example 4, step i), followed by purifying the reaction product with a silica gel column chromatography (chloroform : methanol = 70 : 1) to obtain 318 mg of N-[trans-3-(t-butyldimethylsilyloxy)-6-cyano-3,4-dihydro-2,2-dimethyl-2H-1-benzopyran-4-yl]-N'-hydroxy-3-pyridinecarboxamidine.

$^1$H NMR ($CDCℓ_3$/ppm) δ 0.19(s, 3H), 0.22(s, 3H), 1.00(s, 9H), 1.13(s, 3H), 1.48(s, 3H), 3.75(d, J=10Hz, 1H), 4.44(dd, J=10Hz, 11Hz, 1H), 5.54(d, J=11Hz, 1H), 6.79(d, J=8.4Hz, 1H), 7.3-7.4(2H), 7.47(1H), 8.08(1H), 8.69(1H), 8.99(1H).

ii) 234 mg of N-[trans-3-(t-butyldimethylsilyloxy)-6-cyano-3,4-dihydro-2,2-dimethyl-2H-1-benzopyran-4-yl]-N'-hydroxy-3-pyridinecarboxamidine was desilylated as in Example 4, step ii). Then, the desilylated product was subjected to a silica gel column chromatography (chloroform : methanol = 20 : 1), followed by crystallization from ethyl acetate to give 157 mg of a white solid. Further, the white solid was recrystallized from chloroform-methanol to yield 80 mg of N-(trans-6-cyano-3,4-dihydro-2,2-dimethyl-3-hydroxy-2H-1-benzopyran-4-yl)-N'-hydroxy-3-pyridinecarboxamidine.

Melting point: 124.4 - 126.7 °C

$^1$H NMR ($CDCℓ_3$/ppm) δ 0.92(s, 3H), 1.36(s, 3H), 3.70(dd, J=5Hz, 10Hz, 1H), 4.09(dd, J=10Hz, 10Hz, 1H), 6.01(d, J=5Hz, 1H), 6.35(d, J=10Hz, 1H), 6.87(d, J=8.4Hz, 1H), 7.45(1H), 7.59(1H), 7.75-(1H), 8.10(1H), 8.60(1H), 8.64(1H), 10.11(s, 1H).

Example 6

i) Added to 5.2 mℓ of dry pyridine were 654 mg of 6-cyano-3,4-dihydro-2,2-dimethyl-4-(3-pyridinecarboxamido)-2H-1-benzopyran, which had been prepared from 4-amino-6-cyano-3,4-dihydro-2,2-dimethyl-2H-1-benzopyran described in Published Unexamined Japanese Patent Application No. 2-134357 and nicotinoyl chloride, and 1.56g of phosphorus pentasulfide. The mixture was stirred at 80 °C for 18 hours, followed by cooling. Then, a sodium bicarbonate solution was added to the reaction solution, followed by extraction three times with ethyl acetate. The ethyl acetate layer was washed first with 0.5N hydrochloric acid, then with saline and, finally with a sodium bicarbonate solution. Then, the washed ethyl acetate

layer was dried over mirabilite, followed by removing the solvent by evaporation to give 721g of 6-cyano-3,4-dihydro-2,2-dimethyl-4-(3-pyridinecarbothioamido)-2H-1-benzopyran as a yellow solid.

$^1$H NMR (CDCℓ$_3$/ppm) δ 1.43(s, 3H), 1.52(s, 3H), 1.93(dd, J=11.0Hz, 13.2Hz, 1H), 2.56(dd, J=6.3 Hz, 13.2Hz, 1H), 6.2(m, 1H), 6.81(d, J=8.6Hz, 1H), 7.36(1H), 7.46(1H), 7.60(1H), 8.17(1H), 8.29(br, d, 1H), 8.57(1H), 8.84(s, 1H).

ii) 664 mg of 6-cyano-3,4-dihydro-2,2-dimethyl-4-(3-pyridinecarbothioamido)-2H-1-benzopyran was dissolved in 6.5 mℓ of dry DMF, followed by adding 90 mg of a 60% sodium hydride to the resultant solution while stirring the solution under cooling with ice. The solution was further stirred for 20 minutes, followed by adding 0.15 mℓ of methyl iodide to the solution. The solution was further kept stirred for 1 hour, followed by ice water addition to the reaction solution, extraction three times with ethyl acetate and, then, washing with saline. The ethyl acetate layer was dried and concentrated and, then, purified with a silica gel column chromatography (ethyl acetate: hexane = 1:1) to obtain 591 mg of methyl N-(6-cyano-3,4dihydro-2,2-dimethyl-2H-1-benzopyran-4-yl)-3-pyridinethiocarboximidate.

$^1$H NMR (CDCℓ$_3$/ppm) δ 1.7-2.2(2H), 2.27 and 2.46(s, s, 3H), 4.52 and 5.22(dd, dd, 1H), 6.83 and 6.87(d, d, 1H), 7.3-7.5(3H), 7.71 and 7.94(1H), 8.62 and 8.86(1H), 8.7(1H).

iii) 256 mg of methyl N-(6-cyano-3,4-dihydro-2,2-dimethyl-2H-1-benzopyran-4-yl)-3-pyridine-thiocarboximidate and 140 mg of cyanamide were dissolved in 1 mℓ of dry pyridine, and the resultant solution was kept stirred for 24 hours under a nitrogen gas atmosphere. The solution was further kept stirred for 6 hours at 60°C, followed by adding 50 mg of cyanamide to the solution and an additional stirring for 15 hours under a heated condition. After cooling, water was added to the reaction solution, followed by extraction three times with ethyl acetate and subsequent washing first with 0.1N hydrochloric acid, then with saline and, finally with a sodium bicarbonate solution. The extracts was dried and concentrated, followed by crystallization from ethyl acetate to give 211 mg of N'-cyano-N-(6-cyano-3,4-dihydro-2,2-dimethyl-2H-1-benzopyran-4-yl)-3-pyridinecarboxamidine 1/2 ethyl acetate adduct. The reaction product was further dried at 100°C for 24 hours under a reduced pressure to yield the compound noted above not containing ethyl acetate.

Melting point: 177 - 178°C

$^1$H NMR (CDCℓ$_3$/ppm) δ 1.41(s, 3H), 1.51(s, 3H), 1.90(dd, J=10.9Hz, 13.4Hz, 1H), 2.45(dd, J=6.3Hz, 13.4Hz, 1H), 5.5(m, 1H), 6.79(d, J=8.6Hz, 1H), 7.19(1H), 7.43(1H), 7.48(1H), 7.56(1H), 8.08 (1H), 8.70-(1H), 8.74(s, 1H).

Example 7

286 mg of methyl N-(6-cyano-3,4-dihydro-2,2-dimethyl-2H-1-benzopyran-4-yl)-3-pyridinethiocarboximidate obtained in Example 6, step ii) and 178 mg of hydroxylamine hydrochloride were dissolved in 1.8 mℓ of dry pyridine. The resultant solution was kept stirred for 3 hours at room temperature, followed by evaporating the solvent under a reduced pressure. Water was added to the residue, followed by extraction three times with ethyl acetate. The ethyl acetate layer was washed first with 0.5N hydrochloric acid, then with saline and, finally with a sodium bicarbonate solution. After drying and concentration, the residue thus obtained was subjected to a silica gel column chromatography (ethyl acetate), followed by recrystallization from ethyl acetate to give 86 mg of N-(6-cyano-3,4-dihydro-2,2-dimethyl-2H-1-benzopyran-4-yl)-N'-hydroxy-3-pyridinecarboxamidine.

Melting point: 178.9 - 179.9°C

$^1$H NMR (CDCℓ$_3$/ppm) δ 1.07(s, 3H), 1.42(s, 3H), 1.83(dd, 1H), 2.02(dd, 1H), 4.38(m, 1H), 5.47(d, J=10.8Hz, 1H), 6.62(d, J=8.5Hz, 1H), 7.35-7.55(2H), 7.82(1H), 7.95(1H), 8.71(1H), 8.87(1H).

Example 8

Dissolved in 1 mℓ of dry benzene was 250 mg of 6-cyano-3,4-dihydro-2,2-dimethyl-4-(N-methylacetamido)-2H-1-benzopyran, which had been prepared by the method described in J. Med. Chem., 33,2667 (1990). Then, 75μℓ of phosphorus oxychloride was added to the resultant solution under ice-cooling. The resultant mixture was kept stirred overnight at room temperature, followed by adding 180 mg of aminoacetonitrile. Aminoacetonitrile was further added in an amount of 180 mg 4 hours later and 90 mg additional 3 hours later. The resultant mixture was kept stirred for 1.5 hours. Then, a sodium bicarbonate solution was added to the reaction solution, followed by extraction with ethyl acetate. The organic layer was washed first with a sodium bicarbonate solution, then, with saline, followed by drying over magnesium sulfate. Then, the solvent was removed by evaporation. The residue was roughly refined by a silica gel column chromatography (chloroform : hexane = 9 : 1), followed by further refining with a thin-layer

chromatography to give 66 mg of an oily product, i.e., N-(6-cyano-3,4-dihydro-2,2-dimethyl-2H-1-benzopyran-4-yl)-N'-(cyanomethyl)-N-methylacetamidine.

$^1$H NMR (CDCℓ$_3$/ppm) δ 1.35(s, 3H), 1.48(s, 3H), 1.82(1H), 1.99(dd, J=6.4Hz, 13.1Hz, 1H), 2.10(s, 3H), 2.61(s, 3H), 4.19(s, 2H), 6.0(br, 1H), 6.85(1H), 7.33(1H), 7.41(1H).

Example 9

Dissolved in 2 mℓ of methanol were 113 mg of methyl N-cyano-2-pyridinecarboximidate, which had been prepared by the method described in EP 0388528A, and 131 mg of trans-4-amino-6-cyano-3,4-dihydro-2,2-dimethyl-2H-1-benzopyran-3-ol, which is described in J. Med, Chem., 33,2667 (1990). The resultant solution was kept stirred for 2 hours. After the solvent was evaporated, the residue was subjected to a silica gel column chromatography (chloroform : methanol = 30 : 1), followed by crystallization from methanol to give 144 mg of N'-cyano-N-(trans-6-cyano-3,4-dihydro-2,2-dimethyl-3-hydroxy-2H-1-benzopyran-4-yl)-2-pyridinecarboxamidine.

Melting point: 229.8 - 231.4°C

$^1$H NMR (DMSO-d$_6$/ppm) δ 1.23(s, 3H), 1.43(s, 3H), 3.86(1H), 5.3(1H), 5.97(d, J=5.9Hz, 1H), 6.97(d, J=8.4Hz, 1H), 7.6-7.7(3H), 8.11(d, J=3.7Hz, 1H), 8.78(d, J=5.2Hz, 1H), 9.7(d, 1H).

Example 10

i) From 2.99g of trans-6-cyano-3,4-dihydro-2,2-dimethyl-4-(pyradinecarboxamido)-2H-1-benzopyran-3-ol, which had been prepared by the reaction between trans-4-amino-6-cyano-3,4-dihydro-2,2-dimethyl-2H-1-benzopyran-3-ol and pyradinecarboxylic acid chloride, 4.34g of trans-3-(t-butyldimethylsilyloxy)-6-cyano-3,4-dihydro-2,2-dimethyl-4-(pyradinecarboxamido)-2H-1-benzopyran was obtained according to the procedure described in Example 3, step (i).

$^1$H NMR (CDCℓ$_3$/ppm) δ -0.12(s, 3H), 0.08(s, 3H), 0.81(s, 9H), 1.32(s, 3H), 1.47(s, 3H), 3.86(d, J=8.8Hz, 1H), 5.28(1H), 6.86(d, J=8.4Hz, 1H), 7.4-7.5(2H), 7.95(br d, J=9.9Hz, 1H), 8.54(1H), 8.82(1H), 9.49(1H).

ii) 1g of trans-3-(t-butyldimethylsiliyloxy)-6-cyano-3,4-dihydro-2,2-dimethyl-4-(pyradinecarboxamido)-2H-1-benzopyran and 1.3g of Lawesson's reagent were dissolved in 10 mℓ of toluene. The resultant solution was kept stirred at 120°C for 2.5 hours, followed by cooling the reaction solution. Then, water was added to the solution, followed by extraction with ethyl acetate. The extracts was washed with saline, followed by drying and evaporating the solvent. The residue thus obtained was subjected to a silica gel column chromatography (ethyl acetate : hexane = 1 : 2), followed by refining again with a silica gel column chromatography (chloroform) to give 643 mg of trans-3-(t-butyldimethylsilyloxy)-6-cyano-3,4-dihydro-2,2-dimethyl-4-(pyradinecarbothioamido)-2H-1-benzopyran.

$^1$H NMR (CDCℓ$_3$/ppm) δ -0.15(s, 3H), 0.10(s, 3H), 0.81(s, 9H), 1.38(s, 3H), 1.50(s, 3H), 4.03(1H), 6.17(1H), 6.90(d, J=6.9Hz, 1H), 7.4-7.5(2H), 8.45(1H), 8.22(1H), 9.9.

iii) The reaction of 502 mg of trans-3-(t-butyldimethylsilyloxy)6-cyano-3,4-dihydro-2,2-dimethyl-4-(pyradinecarbothioamido)-2H-1-benzopyran was proceeded as in Example 3, step iii) to give 502 mg of methyl N-[trans-3-(tbutyldimethylsilyloxy)-6-cyano-3,4-dihydro-2,2-dimethyl-2H-1-benzopyran-4-yl] pyradinethiocarboximidate.

iv) 440 mg of methyl N-[trans-3-(t-butyldimethylsilyloxy)-6-cyano-3,4-dihydro-2,2-dimethyl-2H-1-benzopyran-4-yl] pyradinethiocarboximidate was reacted with cyanamide as in Example 3, step iv). Then, the reaction product was refined with a silica gel column chromatography (chloroform : methanol = 99 : 1) to give 414 mg of N-[trans-3-(t-butyldimethylsilyloxy)-6-cyano-3,4-dihydro-2,2-dimethyl-2H-1-benzopyran-4-yl]-N'-cyanopyradinecarboxamidine.

$^1$H NMR (DMSO-d$_6$/ppm) δ -0.07(s, 3H), 0.10(s, 3H), 0.84(s, 9H), 1.28(s, 3H), 1.42(s, 3H), 4.12(d, J=7.8Hz, 1H), 5.4(br, 1H), 7.01(d, J=8.5Hz, 1H), 7.69(d, J=8.5Hz, 1H), 7.88(1H), 8.91(1H), 8.95(1H), 9.30(1H), 9.9(1H).

v) 350 mg of N-[trans-3-(t-butyldimethyl--silyloxy)-6-cyano-3,4-dihydro-2,2-dimethyl-2H-1-benzopyran-4-yl]-N'-cyanopyradinecarboxamidine was processed as in Example 3, step v). Then, the reaction product was refined with a silica gel column chromatography (chloroform : methanol = 50 : 1) to afford 180 mg of N'-cyano-N-(trans-6-cyano-3,4-dihydro-2,2-dimethyl-3-hydroxy-2H-1-benzopyran-4-yl)-pyradinecarboxamidine. Further, the product was recrystallized from chloroform to give 90 mg of crystals of the product compound.

Melting point: 199.0 - 200.6°C

$^1$H NMR (acetone-d$_6$/ppm) δ 1.36(s, 3H), 1.53(s, 3H), 4.11(d, J=9.5Hz, 1H), 5.7(br, 1H), 6.97(d,

J = 8.5Hz, 1H), 7.58(1H), 7.77(s, 1H), 8.78(s, 1H), 8.90(d, J = 2.4Hz, 1H), 9.56(s, 1H).

Example 11

i) 3.19g of methyl 2-pyridinecarboximidate described in EP0388528A was dissolved in 30 mℓ of methylene chloride. The resultant solution was cooled to -5°C, followed by adding 3 mℓ of 2,6-lutidine and subsequently adding dropwise 1.87g of acetyl chloride (solution in 8 mℓ methylene chloride) to the solution at the same temperature. The resultant mixture was kept stirred at room temperature for 3 hours, followed by adding 100 mℓ of methylene chloride to the mixture. Further, the resultant solution was washed first with a dilute citric acid and, then, with water. The organic layer was dried over magnesium sulfate, and the solvent was removed by evaporation to give 3.60g of methyl N-acetyl-2-pyridinecarboximidate.

$^1$H NMR (CDCℓ$_3$/ppm) δ 2.38(s, 3H), 3.94(s, 3H), 7.4(1H), 7.75-7.9(2H), 8.65(d, J = 4.4Hz, 1H).

ii) Added to 1 mℓ of methylene chloride were 51 mg of methyl N-acetyl-2-pyridinecarboximidate and 53mg of 4-amino-6-cyano-3,4-dihydro-2,2-dimethyl-2H-1-benzopyran, followed by adding methanol to the mixture until these added compounds were completely dissolved. Then, 5 mg of sodium methylate was added to the solution, followed by stirring the solution at room temperature for 18 hours. Further, 30 mℓ of methylene chloride was added to the solution, followed by washing with dilute citric acid and water. The organic layer was dried over magnesium sulfate, followed by removing the solvent by evaporation. The residue was roughly refined with a silica gel column chromatography (chloroform : methanol = 50 : 1), followed by crystallization from ether to afford 14 mg of N'-acetyl-N-(6-cyano-3,4-dihydro-2,2-dimethyl-2H-1-benzopyran-4-yl)-2-pyridinecarboxamidine in the form of an isomer mixture.

$^1$H NMR (CDCℓ$_3$/ppm) δ 1.39 1.49 1.52 and 1.62(s, s, s, s, 6H), 1.87 and 2.02 (1H), 2.30 and 2.32(s, s, 3H), 2.3-2.5(1H), 5.07 and 5.29(1H), 6.87(1H).

Example 12

i) 202 mg of methyl N-acetyl-2-pyridinecarboximidate obtained in Example 11, step i) was dissolved in 2 ml of methanol, followed by adding 157 mg of trans-4-amino-6-cyano-3,4-dihydro-2,2-dimethyl-2H-1benzopyran-3-ol to the resultant solution at -10°C. The resultant mixture was kept stirred for one day at room temperature, followed by adding 114 mg of the imidate noted above. The resultant mixture was further kept stirred for one day, followed by removing the solvent by evaporation. Then, chloroform was added to the residue, followed by washing with a dilute citric acid and saline. The extraction liquid was dried and concentrated, followed by crystallization from methanol to give 47 mg of N'-acetyl-N-(trans-6-cyano-3,4-dihydro-2,2-dimethyl-3-hydroxy-2H-1-benzopyran-4-yl)-2-pyridinecarboxamidine.

Melting point: 176.8 - 178.2°C

$^1$H NMR (CD$_3$OD/ppm) δ 1.36(s, 3H), 1.55(s, 3H), 2.24(s, 3H), 3.83(d, J = 8.5Hz, 1H), 6.90(d, J = 8.5Hz, 1H), 7.5-7.6(2H), 7.65(1H), 7.8-8.0(2H), 8.6(1H).

Example 13

i) 2.26g of methyl 2-pyridinecarboximidate was dessolved in 20 mℓ of methylene chloride. After the resultant solution was cooled to -10°C, 1.7 mℓ of ethyl chloroformate was added dropwise to the solution. The solution was stirred for 10 minutes, then, 1.6 mℓ of pyridine was added dropwise to the solution at -20°C, followed by stirring for an hour at the same temperature. After stirring for additional 3 hours at room temperature, the reaction solution was added with methylene chloude, followed by washing with saline. After the washed solution was dried over magnesium sulfate, the solvent was evaporated off to give 2.73g of methyl N-ethoxycarbonyl-2-pyridinecarboximidate in a form of white crystal

$^1$H NMR (CDCℓ$_3$/ppm) δ 1.34(t, J = 7.1Hz, 3H), 3.97(s, 3H), 4.32(q, J = 7.1Hz, 2H), 7.4(1H), 7.8-7.9-(2H), 8.60(1H).

ii) Suspended in 1 mℓ of THF were 148 mg of methyl N-ethoxycarbonyl-2-pyridinecarboximidate, 200 mg of 4-amino-6-cyano-3,4-dihydro-2,2-dimethyl-2H-1-benzopyran and 12 mg of sodium methylate. The resultant suspension was kept stirred at room temperature for 7 days. After the solvent was removed by evaporation, chloroform was added to the residue, followed by removing the non-soluble materials by filtration. Then, the filtrate was washed with a dilute citric acid and saline water. The washed filtrate was dried. The residue obtained by removing the solvent by evaporation was subjected to a silica gel column chromatography (chloroform : methanol = 99 : 1), followed by crystallization from ether to yield 49 mg

of N-(6-cyano-3,4-dihydro-2,2-dimethyl-2H-1-benzopyran-4-yl)-N'-ethoxycarbonyl-2-pyridinecarboxamidine.

Melting point: 124.6 - 126 °C

$^1$H NMR(CDCℓ$_3$/ppm) δ 1.30(t, 3H), 1.38(s, 3H), 1.49(s, 3H), 1.87(dd, 1H), 2.46(dd, 1H), 4.21(q, 2H), 5.37(m, 1H), 6.87(d, J = 8.6Hz, 1H), 7.4-7.9(5H), 8.59(d, J = 4.7Hz, 1H).

Example 14

i) 2.5g of methyl 2-pyridinecarboximidate was dissolved in 50 mℓ of acetonitrile, followed by adding dropwise at -40 °C 12.5 mℓ of acetyl nitrate prepared by the reaction between 22 mℓ of acetic anhydride and 9 mℓ fuming nitric acid. The resultant solution was warmed to -10 °C over 2 hours, followed by removing the solvent by evaporation. Ethyl acetate was added to the residue, followed by washing with a sodium bicarbonate solution and saline. After drying, ethyl acetate was removed by evaporation to give 1.56g of methyl N-nitro-2-pyridinecarboximidate.

$^1$H NMR (CDCℓ$_3$/ppm) δ 4.06(s, 3H), 7.48(1H), 7.78(1H), 7.86(1H), 8.67(1H).

ii) 507 mg of methyl N-nitro-2-pyridinecarboximidate was dissolved in 5 mℓ of benzene, followed by adding 459 mg of trans-4-amino-6-cyano-3,4-dihydro-2,2-dimethyl-2H-1-benzopyran-3-ol to the resultant solution under ice-cooling. The resultant mixture was stirred at room temperature for 50 minutes. Then, the precipitated crystals were separated by filtration, followed by recrystallizatoin from benzene-hexane to give 150 mg of N-(trans-6-cyano-3,4-dihydro-2,2-dimethyl-3-hydroxy-2H-1-benzopyran-4yl)-N'-nitro-2-pyridinecarboxamidine.

Melting point: 133.8 - 137.2 °C

$^1$H NMR (CDCℓ$_3$-CD$_3$OD/ppm) δ 1.12, 1.33 and 1.56(s, s, s, 6H), 3.79(d, 1H), 5.12 and 5.24(d, d, 1H), 6.89 and 6.91(d, d, 1H), 7.3-8.1(m), 8.67(H).

Example 15

Dissolved in 2 mℓ of methanol were 198 mg of methyl N-cyano-4-pyridinecarboximidate described in EP 0388528A and 276 mg of trans-4-amino-6-cyano-3,4-dihydro-2,2-dimethyl-2H-1-benzopyran-3-ol. The resultant solution was kept stirred for 4 days at room temperature. After the solvent was removed by evaporation, the reaction product was subjected to a silica gel column chromatography (chloroform : methanol = 93 : 7) to give 300 mg of N'-cyano-N-(trans-6-cyano-3,4-dihydro-2,2-dimethyl-3-hydroxy-2H-1-benzopyran-4-yl)-4-pyridinecarboxamidine.

$^1$H NMR (CD$_3$OD/ppm) δ 1.37(s, 3H), 1.50(s, 3H), 3.85(d, J = 9.4Hz, 1H), 5.41(d, J = 9.4Hz, 1H), 7.00(d, J = 8.6Hz, 1H), 7.60(1H), 7.22(1H), 7.8(2H), 8.8(2H).

Example 16

Dissolved in 2 mℓ of methanol were 108 mg of methyl N-cyano-4-fluorobenzimidate described in EP 0314446A and 112 mg of trans-4-amino-6-cyano-3,4-dihydro-2,2-dimethyl-2H-1-benzopyran-3-ol. The resultant solution was kept stirred at room temperature for 6 days. After the solution was concentrated, methylene chloride was added to the residue, followed by washing with saline. The washed solution was dried over magnesium sulfate, followed by removing the solvent by evaporation. The residue was subjected to a silica gel column chromatography (chloroform : methanol = 95 : 5) to give 145 mg of N'-cyano-N-(trans-6-cyano-3,4-dihydro-2,2-dimethyl-3-hydroxy-2H-1-benzopyran-4-yl)-4-fluorobenzamidine.

$^1$H NMR (CDCℓ$_3$/ppm) δ 1.30(s, 3H), 1.51(s, 3H), 3.78(d, J = 9.6Hz, 1H), 4.1(br, 1H), 5.27(dd, 1H), 6.8-7.0(2H), 7.1-7.2(2H), 7.4-7.5(2H), 7.6-7.7(1H).

Example 17

Reaction was carried out as in Example 9 between 291 mg of methyl N-cyano-3-pyridinecarboximidate and 284 mg of (3S, 4R)-4-amino-6-cyano-3,4-dihydro-2,2-dimethyl-2H-1-benzopyran-3-ol. The reaction product was purified with a silica gel column chromatography (benzene : ethanol = 9 : 1) to give 393 mg of (-)-N'-cyano-N-[(3S, 4R)-6-cyano-3,4-dihydro-2,2-dimethyl-3-hydroxy-2H-1-benzopyran-4-yl]-3-pyridinecarboxamidine.

$[\alpha]D^{25}$ = -166.9 ° (c = 1. 02; methanol)

Example 18

0.4 mℓ of dry ethanol was added under an argon atmosphere to a mixture consisting of 995 mg of Ethyl N-acetyl-acetyl-4-nitrobenzimidate, which had been prepared from ethyl 4-nitrobenzimidate hydrochloride as in Example 11, step i), and 399 mg of trans-4-amino-6-cyano-3,4-dihydro-2,2-dimethyl-2H-1-benzopyran-3-ol. The mixture was kept stirred at room temperature for 17 hours, followed by adding 10 mℓ of ethanol to the mixture. The mixture was further kept stirred for 1.5 hours. Then, the insolubles were filtrated, followed by crystallization twice from ethanol-methylene chloride to give 237 mg of N'-acetyl-N-(trans-6-cyano-3,4-dihydro-2,2-dimethyl-3-hydroxy-2H-1-benzopyran-4-yl)-4-nitrobenzamidine.

Melting point: 228.8 - 228.7 °C (decomposition)

$^1$H NMR(DMSO-$d_6$/80 °C/ppm) δ 1.28(s, 3H), 1.46(s, 3H), 2.08(s, 3H), 3.70(d, 1H), 4.91(br, 1H), 6.92(d, J = 9Hz, 1H), 7.44(br, 1H), 7.54(d, J = 9Hz, 1H), 7.9(br, 2H), 8.23(d, J = 6Hz, 2H).

Example 19

i) 2.04g of ethyl 4-nitrobenzimidate hydrochloride was dissolved in 15 mℓ of methylene chloride, followed by adding 1.8 mℓ of pyridine to the resultant solution under ice-cooling. Then, 1.3 mℓ of ethyl chloroformate was added dropwise into the solution at the same temperature, followed by stirring the resultant solution at room temperature for 4 hours. During the stirring operation, 10 mℓ of methylene chloride was added to the solution so as to facilitate the stirring which would otherwise be obstructed by the salt precipitated during the stirring operation. After the stirring operation, methlene chloride was added to the reaction mixture, followed by washing with a dilute citric acid solution and saline. After drying over magnesium sulfate, the solvent was removed by evaporation, followed by subjecting the residue to a silica gel column chromatography (chloroform : hexane = 4 : 1) to give 1.94g of ethyl N-ethoxycarbonyl-4-nitrobenzimidate.

$^1$H NMR (CDCℓ$_3$/ppm) δ 1.22(t, J = 7.1Hz, 3H), 1.44(t, J = 7.1Hz, 3H), 4.17(q, J = 7.1Hz, 2H), 4.39(q, J = 7.1Hz, 2H), 7.82(d, J = 9Hz, 2H), 8.27(d, J = 9Hz, 2H).

ii) 128 mg of trans-4-amino-6-cyano-3,4-dihydro-2,2-dimethyl-2H-1-benzopyran-3-ol was added to 151 mg of ethyl N-ethoxycarbonyl-4-nitro-benzimidate in 1 mℓ of ethanol. The resultant mixture was kept stirred at room temperature for 6 days, followed by additional stirring at 50 °C for 24 hours and at 70 °C for 24 hours. Then, the solvent was removed by evaporation. Chloroform was added to the residue, followed by washing with a dilute citric acid solution, with a sodium bicarbonate solution and, then, with saline. After drying the solution over magnesium sulfate, the solvent was removed by evaporation. The residue was subjected to a silica gel column chromatography (ethyl acetate : hexane = 3 : 7), followed by crystallization from ethyl acetate-hexane and, then, from ethyl acetate-ether to give 113 mg of N-(trans-6-cyano-3,4-dihydro-2,2-dimethyl-3-hydroxy-2H-1-benzopyran-4-yl)-N'-ethoxycarbonyl-4-nitrobenzamidine.

Melting point: 139.0 - 141.4 °C

$^1$H NMR (DMSO-$d_6$/ppm) δ 1.01(t, J = 7Hz, 3H), 1.23(s, 3H), 1.45(s, 3H), 3.75(dd, 1H), 3.86(q, J = 7Hz, 2H), 5.13(dd, 1H), 6.04(d, J = 5.7Hz, 1H), 6.96(d, J = 8.4Hz, 1H), 7.6-7.7(2H), 7.81(d, J = 8.7Hz, 2H), 8.33-(d, J = 8.7Hz, 2H), 8.73(d, J = 8.3Hz, 1H).

Example 20

i) Dissolved in 16 mℓ of dry pyridine was 2.03g of 4-(acetamido)-6-cyano-3,4-dihydro-2,2-dimethyl-2H-1-benzopyran, which had been prepared by the method described in J. Med. Chem., 33,2667 (1990). Then, 5.71g of phosphorus pentasulfide was added to the resultant solution while stirring the solution, followed by further stirring at 80 °C for 1.5 hours. The reaction solution was concentrated under a reduced pressure, followed by addition of a sodium bicarbonate solution and, then, extraction three times with ethyl acetate. The ethyl acetate layer was washed with 0.5N hydrochloric acid, saline and sodium bicarbonate solution. After drying, the solvent was removed by evaporation to give 1.76g of 6-cyano-3,4-dihydro-2,2-dimethyl-4-(thioacetamido)-2H-1-benzopyran.

$^1$H NMR (CDCℓ$_3$/ppm) δ 1.38(s, 3H), 1.48(s, 3H), 1.75(dd, J = 11.6Hz, 13.3Hz, 1H), 2.45(dd, J = 6.3Hz, 13.3Hz, 1H), 2.66(s, 3H), 6.03(m, 1H), 6.87(d, J = 10.0Hz, 1H), 7.3-7.5(2H), 7.54(1H).

ii) 1.52g of 6-cyano-3,4-dihydro-2,2-dimethyl-4-(thioacetamido)-2H-1-benzopyran was dissolved in 16 mℓ of dry DMF, followed by adding 270 mg of a 60% sodium hydride to the resultant solution while stirring the solution. After hydrogen gas ceased to be generated, 0.42 ml of methyl iodide was added to the solution, followed by stirring at room temperature for 1.5 hours. Water was added to the reaction solution,

followed by extraction with ethyl acetate. Then, the extract was washed with saline, followed by drying over merabilite. After removal of the solvent by evaporation, the residue was subjected to a silica gel column chromatography (ethyl acetate : hexane = 1 : 4) to obtain 0.99g of methyl N-(6-cyano-3,4-dihydro-2,2-dimethyl-2H-1-benzopyran-4-yl)thioacetimidate in the form of an isomer mixture.

$^1$H NMR (CDCℓ$_3$/ppm) δ 1.37 and 1.39(s, s, 3H), 1.46 and 1.48(s, s, 3H), 1.7-2.1(m, 2H), 2.21, 2.29, 2.42 and 2.56(s, s, s, s, 6H), 4.74 and 4.85(dd, dd, 1H), 6.82 and 6.84(d, d, 1H), 7.26 and 7.38(1H), 7.4-(1H).

iii) 543 mg of methyl N-(6-cyano-3,4-dihydro-2,2-dimethyl-2H-1-benzopyran-4-yl) thioacetimidate was dissolved in 5 mℓ of pyridine, followed by adding 280 mg of hydroxylamine hydrochloride to the resultant solution. The resultant mixture was kept stirred at room temperature for 3 hours, followed by removing pyridine by distillation. The residue was subjected to a silica gel column chromatography (chloroform : methanol = 50 : 1) to give 440 mg of N-(6-cyano-3,4dihydro-2,2-dimethyl-2H-1-benzopyran-4-yl)-N'hydroxyacetamidine.

$^1$H NMR (CDCℓ$_3$/ppm) δ 1.35(s, 3H), 1.48(s, 3H), 1.81(dd, J = 11.6Hz, 13.4Hz, 1H), 2.02(s, 3H), 2.15-(dd, J = 5.8Hz, 13.4Hz, 1H), 4.6(m, 1H), 5.4(1H), 6.64(d, J = 8.5Hz, 1H), 7.45(1H), 7.70(1H).

Example 21

i) 4.04g of ethyl acetimidate hydrochloride and 12 mℓ of triethylamine were added to 40 mℓ of methylene chloride, followed by adding dropwise 4 mℓ of ethyl chloroformate (solution in 10 mℓ of methylene chloride) while stirring. The mixture was kept stirred for 2 days. Then, water was added to the reaction solution, followed by extraction twice with methylene chloride. The extracts was washed with saline, followed by drying over magnesium sulfate. Then, the solvent was removed by evaporation to give 2.57g of ethyl N-ethoxycarbonylacetimidate.

$^1$H NMR (CDCℓ$_3$/ppm) δ 1.29(t, J = 7Hz, 3H), 1.31(t, J = 7Hz, 3H), 2.10(s, 3H), 4.16(q, J = 7Hz, 2H), 4.19(q, J = 7Hz, 2H).

ii) 0.4 mℓ of dry ethanol was added to a mixture consisting of 350 mg of ethyl N-ethoxycarbonylacetimidate and 400 mg of trans-4-amino-6-cyano-3,4-dihydro-2,2-dimethyl-2H-1-benzopyran-3-ol. The resultant mixture was stirred at room temperature. Further, 349 mg of said imidate was added to the reaction solution 4 days later, followed by stirring the mixture for 2 days. Then, the solvent was removed by evaporation. The residue was subjected to a silica gel column chromatography (ethyl acetate : hexane = 2 : 1), followed by crystallization from ether to yield 101 mg of N-(trans-6-cyano-3,4-dihydro-2,2-dimethyl-3-hydroxy-2H-1-benzopyran-4-yl)-N'ethoxycarbonylacetamidine.

Melting point: 152 ° C < (decomposition)

$^1$H NMR (DMSO-d$_6$/ppm) δ 1.81(s, 3H), 1.19(t, J = 7Hz, 3H), 1.41(s, 3H), 2.23(s, 3H), 3.60(d, 1H), 4.00(q, J = 7Hz, 2H), 5.06(dd, 1H), 5.84(1H), 6.94(d, J = 8.5Hz, 1H), 7.52(1H), 7.62(1H), 9.31(1H).

Example 22

Reaction was carried out as in Example 16 between 502 mg of methyl N-cyano-4-fluorobenzimidate and 625 mg of (3S, 4R)-4-amino-6-cyano-3,4-dihydro-2,2-dimethyl-2H-1-benzopyran-3-ol. The reaction product was refined by a silica gel column chromatography (chloroform : methanol = 97 : 3) to give 928 mg of (-)-N'-cyano-N-[(3S, 4R)-6-cyano-3,4-dihydro-2,2-dimethyl-3-hydroxy-2H-1-benzopyran-4-yl]-4-fluorobenzamidine, which is the levorotatory optically active isomer of the compound obtained in Example 16.

$[\alpha]_D^{25}$ = -149.1 ° (c = 1.02; methanol)

Example 23

Added to 2 mℓ of ethanol were 415 mg of ethyl N-cyano-4-nitrobenzimidate, which is prepared by the method substantially same as described in EP 0388528A, and 345 mg of trans-4-amino-6-cyano-3,4-dihydro-2,2-dimethyl-2H-1-benzopyran-3-ol. The mixture was kept stirred for 9 days. After the solvent was evaporated, the reaction product was subjected to a silica gel column chromatography (chloroform : methanol = 97 : 3), followed by crystallization from ethyl acetate-ether to afford 300 mg of N'-cyano-N-(trans-6-cyano-3,4-dihydro-2,2-dimethyl-3-hydroxy-2H-1-benzopyran-4-yl)-4-nitrobenzamidine.

Melting point: 171 - 173 ° C

$^1$H NMR (DMSO-d$_6$/ppm) δ 1.24(s, 3H), 1.44(s, 3H), 3.71(dd, 1H), 5.19(dd, 1H), 6.12(d, J = 5.6Hz, 1H), 6.97(d, J = 8.5Hz, 1H), 7.65(d, J = 8.5Hz, 1H), 7.87(s, 1H), 8.05(d, J = 8.6Hz, 2H), 8.45(d, J = 8.6Hz, 2H), 9.70-(d, J = 8.2Hz, 1H).

Example 24

Added to 3 mℓ of ethanol were 508 mg of ethyl N-cyano-4-nitrobenzimidate and 508 mg of (3S, 4R)-4-amino-6-cyano-3,4-dihydro-2,2-dimethyl-2H-1-benzopyran-3-ol. The mixture was kept stirred for 4 days. Then, 101 mg of said imidate was further added to the mixture. The resultant mixture was kept stirred for 7 days, followed by removing the solvent by evaporation. The residue was subjected to a silica gel column chromatography (chloroform : methanol = 97 : 3), followed by crystallization from ethyl acetate-ether to give 307 mg of (-)-N'-cyano-N-[(3S, 4R)-6-cyano-3,4-dihydro-2,2-dimethyl-3-hydroxy-2H-1-benzopyran-4-yl)-4-nitrobenzamidine, which is the levorotatory optical by active isomer of the compound obtained in Example 23.

Melting point: 156 - 158 ° C

$[\alpha]_D^{25}$ = -138.4 ° (c = 1.02; methanol)

Further, 409 mg of the compound noted above was recovered by adding ether to the mother liquor.

Example 25

1 mℓ of methylene chloride was added to a mixture consisting of 730 mg of ethyl N-acetyl-4-fluorobenzimidate, which had been prepared from ethyl 4-fluorobenzimidate hydrochloride as in Example 1, step i), and 611 mg of trans-4-amino-6-cyano-3,4-dihydro-2,2-dimethyl-2H-1-benzopyran-3-ol. Further, ethanol was added to the mixture until all the reactants were completely dissolved. The resultant solution was kept stirred for 21 hours at room temperature, followed by separating the precipitated solid material by filtration. The solid was refined with a silica column chromatography (ethyl acetate : hexane = 6 : 4). A white powder thus obtained was washed with ethanol to give 282 mg of N'-acetyl-N-(trans-6-cyano-3,4-dihydro-2,2-dimethyl-3-hydroxy-2H-1-benzopyran-4-yl)-4-fluorobenzamidine.

Melting point: 198 ° C < (decomposition)

$^1$H NMR ($CD_3OD$/ppm) δ 1.36(s, 3H), 1.56(s, 3H), 2.20(s, 3H), 3.80(d, J = 9.3Hz, 1H), 5.1(br, 1H), 6.97(d, 1H), 7.23(t, 2H), 7.55(dd, 1H), 7.66(s, 1H), 7.72-7.87(2H).

Example 26

2 mℓ of methylene chloride was added to a mixture consisting of 679 mg of ethyl N-ethoxycarbonyl-4-fluorobenzimidate, which had been prepared from ethyl 4-fluorobenzimidate hydrochloride as in Example 13, step i), and 549 mg of trans-4-amino-6-cyano-3,4-dihydro-2,2-dimethyl-2H-1-benzopyrane-3-ol. Further, ethanol was added to the mixture until all the reactants were completely dissolved. The resultant solution was kept stirred for 4 days at room temperature, followed by further adding 2 mℓ of ethanol. The resultant solution was kept stirred for 1 day at 50 ° C, followed by removing the solvent by evaporation under a reduced pressure. The powdery material thus obtained was washed first with methanol and, then, with water to give 270 mg of N-(trans-6-cyano-3,4-dihydro-2,2-dimethyl-3-hydroxy- 2H-1-benzopyran-4-yl)-N'-ethoxycarbonyl-4-fluorobenzamidine.

Melting point: 215 ° C < (decomposition)

$^1$H NMR (CMSO-$d_6$/ppm) δ 1.01(t, J = 7.0Hz, 3H), 1.22(s, 3H), 1.45(s, 3H), 3.75(dd, 1H), 3.87(q, J = 7.0Hz, 2H), 5.08(t, 1H), 5.97(d, 1H), 6.95(d, 1H), 7.33(t, 2H), 7.5-7.7(4H), 8.47(d, 1H).

Example 27

Reaction was carried out as in Example 9 between 306 mg of methyl N-cyano-3-pyridinecarboximidate and 412 mg of (3R, 4S)-4-amino-6-cyano-3,4-dihydro-2,2-dimethyl-2H-1-benzopyran-3-ol. Then, the reaction product was refined with a silica gel column chromatography (benzene : ethanol = 93 : 7) to give 462 mg of (+)-N'-cyano-N-[(3R, 4S)-6-cyano-3,4-dihydro-2,2-dimethyl-3-hydroxy-2H-1-benzopyran-4-yl]-3-pyridinecarboxamidine, which is the dextrorotatory optically active isomer of the compound obtained in Example 3.

$[\alpha]_D^{25}$ = +172.4 ° (c = 0.985; methanol)

Example 28

503 mg of N'-cyano-N-(trans-6-cyano-3,4-dihydro-2,2-dimethyl-3-hydroxy-2H-1-benzopyran-4-yl)-3-pyridinecarboxamidine, which was obtained in Example 3, was dissolved in 3 mℓ of pyridine, followed by adding dropwise 0.45 mℓ of acetic anhydride to the resultant solution under cooling with ice. The resultant

solution was kept stirred for 30 minutes. The solution was further stirred at room temperature for 6 hours, followed by removing the solvent by evaporation under a reduced pressure. Ethyl acetate was added to the residue, followed by washing first with a 10% citric acid, then, with a sodium bicarbonate solution and, finally, with saline. The organic layer was dried over magnesium sulfate, followed by evaporating off the solvent to give 660 mg of N'-cyano-N-(trans-3-acetoxy-6-cyano-3,4-dihydro-2,2-dimethyl-2H-1-benzopyran-4-yl]-3-pyridinecarboxamidine.

$^1$H NMR (CDCℓ$_3$/ppm) δ 1.41(s, 3H), 1.45(s, 3H), 5.22(d, J=9.7Hz, 1H), 5.65(1H), 6.94(d, 1H), 7.4-7.6-(3H), 7.90(d, 1H), 8.12(1H), 8.62(1H), 8.77(1H).

Example 29

Dissolved in 0.5 mℓ of methanol were 172 mg of methyl N-cyano-3-pyridinecarboximidate and 209 mg of trans-4-amino-3,4-dihydro-2,2-dimethyl-6-fluoro-2H-1-benzopyran-3-ol which is described in EP 0412531A while stirring the solution at room temperature. Methyl N-cyano-3-pyridinecarboximidate was added to the solution in an amount of 92 mg 1 day later and 117 mg 4 days later. After stirring the solution for 7 days in total, the solvent was removed by evaporation. Ethyl acetate was added to the residue, followed by washing with a 10% citric acid and saline and, then, drying over magnesium sulfate. The residue obtained by removing the solvent was refined with a silica gel column chromatography (chloroform : methanol = 9 : 1) to give 261 mg of N'-cyano-N-(trans-3,4-dihydro-2,2-dimethyl-6-fluoro-3-hydroxy-2H-1-benzopyran-4-yl)-3-pyridinecarboxamidine.

$^1$H NMR (DMSO-d$_6$/ppm) δ 1.17(s, 3H), 1.41(s, 3H), 3.71(dd, J=5.6Hz, 9.3Hz, 1H), 5.14(t, 1H), 5.98(d, J=5.6Hz, 1H), 6.80(dd, 1H), 7.00-7.15(2H), 7.64(dd, 1H), 8.20(dt, 1H), 8.80(dd, 1H), 8.93(d, 1H), 9.70(d, 1H).

Example 30

Dissolved in 0.6 mℓ of methanol were 355 mg of methyl N-cyano-3-pyridinecarboximidate and 234 mg of trans-4-amino-3,4-dihydro-2,2-dimethyl-6-ethyl-2H-1-benzopyran-3-ol which had been prepared by the method substantially same as described in J. Med, Chem., 33,3023 (1990), while stirring the solution at room temperature. Methyl N-cyano-3-pyridinecarboximidate was added to the solution in an amount of 149 mg 2 days later, and the solution was further kept stirred for 5 days. Then, the solution was stirred for 1 day at 60°C, followed by removing the precipitate by filtration. The filtrate was, then, concentrated and ethyl acetate was added to the residue, followed by washing first with a 10% aqueous solution of citric acid and, then, with saline. After drying over magnesium sulfate, the solvent was removed by evaporation. The residue thus obtained was subjected to a silica gel column chromatography (chloroform : methanol = 9 : 1) to give 153 mg of N'-cyano-N-(trans-3,4-dihydro-2,2-dimethyl-6-ethyl-3-hydroxy-2H-1-benzopyrane-4-yl)-3-pyridinecarboxamidine.

$^1$H NMR (DMSO-d$_6$/ppm) δ 1.13(t, J=7.6Hz, 3H), 1.17(s, 3H), 1.40(s, 3H), 2.5(2H), 3.69(dd, J=5.6Hz, 9.3Hz, 1H), 5.15(t, 1H), 5.91(d, J=5.6Hz, 1H), 6.71(d, 1H), 7.00(2H), 7.64(dd, 1H), 8.16(d, 1H), 8.80(d, 1H), 8.90(d, 1H), 9.65(d, 1H).

Example 31

5.35g of trans-4-amino-6-cyano-3,4-dihydro-2,2-dimethyl-2H-1-benzopyran-3-ol was dissolved in a solvent mixture consisting of 50 mℓ of dioxane and 10 mℓ of water, followed by adding 6.16g of di-t-butyldicarbonate to the resultant solution under ice-cooling. The mixture was kept stirred for 3.5 hours at room temperature, followed by removing the solvent by evaporation. Ethyl acetate was added to the residue, followed by washing with a 10% aqueous solution of citric acid and saline. Then, the solution was dried over magnesium sulfate, followed by removing the solvent by evaporation. 8.78g of crystals thus obtained were dissolved in a mixed solvent consisting of 80 mℓ of acetonitrile and 20 mℓ of chloroform, followed by adding dropwise 8 mℓ of acetylnitrate prepared by the ordinary method into the resultant solution at -30°C. The mixture was kept stirred for 3 hours at -5°C, followed by further adding dropwise 3 mℓ of acetylnitrate into the solution. Then, the resultant mixture was stirred under ice-cooling. An ice-sodium bicarbonate solution mixture was added to the reaction solution 2 hours later, followed by extracting with chloroform and subsequently washing the extract with saline water. The washed extract was dried over magnesium sulfate, followed by removing the solvent by evaporation. The residue was refined with a silica gel column chromatography (chloroform). One gram of crystals, which were taken from the resultant crystals, were dissolved in 10 mℓ of methylene chloride, followed by adding 5 mℓ of a 50% trifluoro-acetic acid/methylene chloride to the resultant solution. The mixture was kept stirred for 1 hour at room

temperature, followed by pouring the reaction solution to a sodium bicarbonate solution and subsequently extracting with chloroform. The extract was washed with a saturated saline, followed by drying over magnesium sulfate. Further, the solvent was removed by evaporation to give crystals of trans-4-amino-6-cyano-3,4-dihydro-2,2-dimethyl-3-nitroxy-2H-1-benzopyran. 219g of the product compound, which was taken from the product obtained, and 126 mg of methyl N-cyano-2-pyridinecarboximidate were dissolved in 0.5 mℓ of methanol. The resultant solution was kept stirred at 70° C for 10 hours. The residue obtained by removing the solvent was subjected to a silica gel column chromatography (ethyl acetate : hexane = 2 : 3), followed by refining with a thin-layer chromatography to give 121 mg of N'-cyano-N-(trans-6-cyano-3,4-dihydro-2,2-dimethyl-3-nitroxy-2H-1-benzopyrane-4-yl)-2-pyridinecarboxamidine.

$^1$H NMR ($CDCℓ_3$/ppm) δ 1.43(s, 3H), 1.57(s, 3H), 5.55(d, 1H), 5.8(br, 1H), 6.99(dd, 1H), 7.5-7.7(3H), 8.01(dt, 1H), 8.6-8.7(2H), 8.88(br, 1H).

Example 32

Added to 0.6 mℓ of ethanol were 299 mg of trans-4-amino-3,4-dihydro-2,2-dimethyl-6-(trifluoromethoxy)-2H-1-benzopyran-3-ol, which is described in Published Unexamined Japanese Patent Application No. 1-151571 and 220 mg of methyl N-cyano-3-pyridinecarboximidate. The resultant mixture was kept stirred for 18 hours. The residue obtained by removing the solvent by evaporation was subjected to a silica gel column chromatography (chloroform : methanol = 9 : 1), followed by crystallization from ethyl acetate-hexane to give 181 mg of N'-cyano-N-[trans-3,4-dihydro-2,2-dimethyl-3-hydroxy-6-(trifluoromethoxy)-2H-1-benzopyran-4-yl]-3-pyridinecarboxamidine.

Melting point: 180.0 - 181.4° C

$^1$H NMR ($CD_3OD$/ppm) δ 1.29(s, 3H), 1.50(s, 3H), 3.79(d, J=9.0Hz, 1H), 3.37(d, J=9.0Hz, 1H), 6.87(d, 1H), 7.12(d, 1H), 7.17(s, 1H), 7.64(dd, 1H), 8.20(ddd, 1H), 8.77(dd, 1H), 8.87(d, 1H).

Example 33

Dissolved in 0.6 mℓ of methanol were 301 mg of trans-4-amino-3,4-dihydro-2,2-dimethyl-6-nitro-2H-1-benzopyran-3-ol, which is described in EP 0412531A, and 254 mg of methyl N-cyano-3-pyridinecarboximidate. The resultant solution was kept stirred at room temperature. 0.6 mℓ of methanol was added to the solution 23 hours later, followed by stirring the solution for 15 hours. A small amount of methanol was added to the reaction solution, followed by heating to dissolve the precipitated material. Then, the solution was cooled to room temperature, followed by separating the precipitated crystals by filtration to give 233 mg of N'-cyano-N-(trans-3,4-dihydro-2,2-dimethyl-3-hydroxy-6-nitro-2H-1-benzopyran-4-yl)-3-pyridinecarboxamidine.

Melting point: 215.7 - 217.7° C

$^1$H NMR (DMSO-$d_6$/ppm) δ 1.28(s, 3H), 1.48(s, 3H), 3.82(d, J=8.4Hz, 1H), 5.23(1H), 6.16(1H), 7.04(d, J=8.9Hz, 1H), 8.05-8.25(m, 3H), 8.83(m, 1H), 9.91(1H), 9.77(d,1H).

Example 34

Added to 0.1 mℓ of ethanol were 145 mg of ethyl N-cyano-2-chloro-3-pyridinecarboximidate, which had been prepared by the method disclosed in EP 0388528A, and 121 mg of trans-4-amino-6-cyano-3,4-dihydro-2,2-dimethyl-2H-1-benzopyran-3-ol. The resultant mixture was kept stirred for 99 hours. Then, the solvent was removed by evaporation. The residue was subjected to a silica gel column chromatography (ethyl acetate : hexane = 9 : 1) and, then, refined with a thin-layer chromatography, followed by crystallization from methylene chloride to give 50 mg of N'-cyano-N-(trans-6-cyano-3,4-dihydro-2,2-dimethyl -3-hydroxy-2H-1-benzopyran-4-yl)-2-chloro-3-pyridinecarboxamidine.

Melting point: 152.2 - 154.2° C

$^1$H NMR ($CD_3OD$/ppm) δ 1.32(s, 3H), 1.50(s, 3H), 3.78(d, J=9.3Hz, 1H), 5.32(d, J=9.3Hz, 1H), 6.32(d, J=8.4Hz, 1H), 7.50-7.65(m, 2H), 7.76(1H), 8.08(1H), 8.57(m, 1H).

Example 35

Added to 0.4 mℓ of methanol were 401 mg of methyl N-cyano-2-thiophenecarboximidate, which had been prepared by the method disclosed in EP 0388528A, and 352 mg of trans-4-amino-6-cyano-3,4-dihydro-2,2-dimethyl-2H-1-benzopyran-3-ol. The resultant mixture was kept stirred for 27 hours. Then, water was added to the reaction solution, followed by extraction with ethyl acetate and, then, washing with a

saturated saline. After drying over magnesium sulfate, the solvent was removed by evaporation, followed by dissolving the residue in a heated methanol. The solution was then cooled, followed by separating crystals by filtration. The crystals thus obtained was recrystallized from ethanol to give 130 mg of N'-cyano-N-(trans-6-cyano-3,4-dihydro-2,2-dimethyl-3-hydroxy-2H-1-benzopyran-4yl)-2-thiophenecarboxamidine.

Melting point: 159.3 - 163.0 °C

$^1$H NMR ($CD_3OD$/ppm) δ 1.30(s, 3H), 1.52(s, 3H), 3.84(d, J = 9.6Hz, 1H), 5.37(d, J = 9.6Hz, 1H), 6.49(d, J = 9.0Hz, 1H), 7.27(m, 1H), 7.53(m, 1H), 7.86(1H), 7.95(m, 1H).

Example 36

0.8 mℓ of ethanol was added to a mixture consisting of 491 mg of ethyl N-cyano-4-(trifluoromethyl)-benzimidate, which had been prepared by the method disclosed in EP0388528A, and 351 mg of trans-4-amino-6-cyano-3,4-dihydro-2,2-dimethyl-2H-1-benzopyran-3-ol. The resultant mixture was kept stirred at room temperature. 36 hours later, 192 mg of ethyl N-cyano-4-(trifluoromethyl)benzimidate was added to the reaction solution, followed by further stirring for 17 hours. The reaction solution was concentrated, followed by water addition to the residue and, then, extraction with ethyl acetate. The organic layer was washed with saline, followed by drying over magnesium sulfate. The residue obtained by removing the solvent by evaporation was subjected to a silica gel column chromatography (ethyl acetate : hexane = 2 : 3), followed by recrystallization from dioxane to give 210 mg of N'-cyano-N-(trans-6-cyano-3,4-dihydro-2,2-dimethyl-3-hydroxy-2H-1-benzopyran-4-yl)-4-(trifluoromethyl)benzamidine.

Melting point: 246 - 247 °C

$^1$H MNR ($CD_3OD$/ppm) δ 1.32(s, 3H), 1.52(s, 3H), 3.81(d, J = 9.3Hz, 1H), 5.38(d, J = 9.3Hz, 1H), 6.59(d, J = 8.6Hz, 1H), 7.55(dd, 1H), 7.65(1H), 7.92(m, 3H).

Example 37

Added to 0.4 mℓ of ethanol were 402 mg of ethyl N-cyano-2-fluorobenzimidate and 350 mg of trans-4-amino-6-cyano-3,4-dihydro-2,2-dimethyl-2H-1-benzopyran-3-ol. The resultant mixture was stirred at room temperature. Then, 204 mg of the imidate and 0.6 mℓ of ethanol were added to the resultant solution the next day. The mixture was kept stirred at room temperature for 5 hours, followed by heating to 50 °C and stirring for 3 hours. The solvent was removed by evaporation, followed by water addition and, then, extraction with ethyl acetate. The extract was washed with saline, followed by drying over magnesium sulfate. Then, the solvent was removed by evaporation, followed by crystallization from ethanol to give 292.5 mg of N'-cyano-N-(trans-6-cyano-3,4-dihydro-2,2-dimethyl-3-hydroxy-2H-1-benzopyran-4-yl)-2-fluorobenzamidine.

Melting point: 128.5 - 128.7 °C

$^1$H MNR (DMSO-$d_6$/ppm) δ 1.24(s, 3H), 1.43(s, 3H), 3.72(m, 1H), 5.15(1H), 6.03(d, J = 5.8Hz, 1H), 6.98(d, J = 8.5Hz, 1H), 7.35-7.55(m, 2H), 7.55-7.85(m, 4H), 9.73(1H).

Example 38

Added to 0.4 mℓ of ethanol were 397 mg of ethyl N-cyano-3-fluorobenzimidate and 350 mg of trans-4-amino-6-cyano-3,4-dihydro-2,2-dimethyl-2H-1-benzopyran-3-ol. The resultant mixture was stirred at room temperature. Then, 204 mg of the imidate and 0.2 mℓ of ethanol were added to the resultant solution 24 hours later, followed by further stirring for 25 hours. The solvent was removed by evaporation, followed by water addition and, then, extraction with ethyl acetate. Further, the extract was washed with saline, followed by drying over magnesium sulfate. The residue obtained by removing the solvent by evaporation was subjected to a silica gel column chromatography (ethyl acetate : hexane = 1 : 2), followed by crystallization from ethanol to give 305.5 mg of N'-cyano-N-(trans-6-cyano-3,4-dihydro-2,2-dimethyl-3-hydroxy-2H-1-benzopyran-4-yl)-3-fluorobenzamidine 1/2 ethanol adduct.

Melting point: 161 - 162 °C

$^1$H NMR (DMSO-$d_6$/ppm) δ 1.23(s, 3H), 1.45(s, 3H), 3.47(m, 1H), 5.16(1H), 6.07(d, J = 5.8Hz, 1H), 6.97(d, J = 8.5Hz, 1H), 7.45-7.55(m, 1H), 7.55-7.85(m, 5H), 9.51(1H).

Example 39

Added to 0.6 mℓ of methanol were 301 mg of trans-4-amino-6-bromo-3,4-dihydro-2,2-dimethyl-2H-1-benzopyran-3-ol, which is described in EP 0412531A, and 220 mg of methyl N-cyano-3-pyridinecarboximidate. The resultant mixture was stirred at room temperature. Then, 104 mg of said imidate and 0.3 mℓ of methanol were added to the resultant solution 3 days later, followed by further stirring for 1 day. The solvent was removed by evaporation, followed by subjecting the residue to a silica gel column chromatography (chloroform : methanol = 95 : 5), followed by crystallization from ethyl acetate-hexane to give 82 mg of N'-cyano-N-(trans-6-bromo-3,4-dihydro-2,2-dimethyl-3-hydroxy-2H-1-benzopyran-4-yl)-3-pyridinecarboxamidine.

Melting point: 219 - 220 ° C

$^1$H NMR ($CD_3OD$/ppm) δ 1.28(s, 3H), 1.48(s, 3H), 3.76(d, J = 9.4Hz, 1H), 5.35(d, J = 9.4Hz, 1H), 6.73(d, J = 8.7Hz, 1H), 7.31(1H), 7.36(1H), 7.65(1H), 8.22(1H), 8.78(1H), 8.89(1H).

Example 40

Added to 1.2 mℓ of ethanol were 759 mg of ethyl N-cyano-3-pyridinecarboximidate and 591 mg of trans-4-amino-3,4-dihydro-2,2-dimethyl-6-methoxy-2H-1-benzopyran-3-ol. The resultant mixture was stirred for 37 hours. Then, the reaction solution was concentrated, followed by subjecting the residue to a silica gel column chromatography (chloroform : methanol = 95 : 5), followed by crystallization from methanol to give 442.7 mg of N'-cyano-N-(trans-3,4-dihydro-2,2-dimethyl-3-hydroxy-6-methoxy-2H-1-benzopyran-4-yl)-3-pyridin-carboxamidine.

Melting point: 119.9 - 122.4 ° C

$^1$H NMR ($CDC\ell_3$/ppm) δ 1.27(s, 3H), 1.45(s, 3H), 3.71(s, 3H), 3.73(1H), 4.40(br, 1H), 5.31(1H), 6.68(1H), 6.73(1H), 6.76 (1H), 7.41(1H), 7.63(1H), 8.02(1H), 8.52(1H), 8.66(1H).

Example 41

Dissolved in a mixed solvent were 364 mg of N'-cyano-N-(trans-6-cyano-3,4-dihydro-2,2-dimethyl-3-hydroxy-2H-1-benzopyran-4-yl)-3-pyridinecarboxamidine, which was obtained in Example 3, and 517 mg of a 70% m-chloroperbenzoic acid, said mixed solvent consisting of 20 mℓ of methylene chloride and 4 mℓ of methanol. The resultant solution was kept stirred for 20 hours at room temperature. Then, ether was added to a solid material obtained by removing the solvent by evaporation, and the solid material was pulverized followed by stirring the mixture. Further, the insoluble solid material was removed by filtration, followed by crystallization from ethanol to give 191 mg of N'-cyano-N-(trans-6-cyano-3,4-dihydro-2,2-dimethyl-3-hydroxy-2H-1-benzopyran-4-yl)-3-pyridinecarboxamidine 1-oxide.

Melting point: at least 200 ° C (decomposition)

$^1$H NMR ($CDC\ell_3$ - $CD_3OD$/ppm) δ 1.33(s, 3H), 1.56(s, 3H), 3.75(s, d, J = 9.8Hz, 1H), 5.32(d, J = 9.8Hz, 1H), 6.91(d, J = 6.91Hz, 1H), 7.47-7.50(2H), 7.64(1H), 8.03(1H), 8.38(1H), 8.51(1H).

Example 42

5 mℓ of benzene and 1.2 mℓ of 1,8-diazabicyclo [5.4.0]-7-undecene were added to 650 mg of N'-cyano-N-(trans-3-acetoxy-6-cyano-3,4-dihydro-2,2-dimethyl-2H-1-benzopyran-4-yl)-3-pyridinecarboxamidine obtained in Example 28. The mixture was kept stirred for 20 hours under a reflux condition. Ethyl acetate was added to the residue obtained by removing the solvent by evaporation, followed by washing with saline. After drying over magnesium sulfate, the solvent was removed by evaporation. Further, the reaction product was refined with a thin-layer chromatography to give 83 mg of N'-cyano-N-(6-cyano-2,2-dimethyl-2H-1-benzopyran-4-yl)-3-pyridinecarboxamidine.

$^1$H NMR (DMSO-$d_6$/ppm) δ 1.49(s, 6H), 6.22(s, 1H), 7.00(d, 1H), 7.6-7.75(2H), 7.82(s, 1H), 8.27(d, 1H), 8.83(d, 1H), 9.04(s, 1H), 10.60(s, 1H).

Example 43

Added to 2.1 mℓ of ethanol were 2.26g of ethyl N-cyano-3-pyridinecarboximidate and 2.06g of t-4-amino-6-cyano-3,4-dihydro-2-methyl-2-propyl-2H-1-benzopyran-r-3-ol, which had been prepared by the method disclosed in Published Unexamined Japanese Patent Application No. 2-184686. The resultant mixture was stirred for 16 hours. Then, the reaction solution was concentrated, followed by subjecting the residue to a silica gel column chromatography (chloroform : methanol = 96 : 4) to separate isomers, followed by crystallization from methanol to give 220.8 mg of a low polarity isomer of N'-cyano-N-(6-cyano-3,4-dihydro-r-3-hydroxy-2-methyl-2-propyl-2H-1-benzopyran-t-4-yl)-3-pyridinecarboxamidine (melting point: 240 ° C decomposition) and 246.1 mg of a high polarity isomer of said compound

Melting point: 242° (decomposition).

$^1$H NMR (DMSO-$d_6$/ppm: low polarity isomer) δ 0.93(t, J=7.2Hz, 3H), 1.23(s, 3H), 1.4-1.6(m, 2H), 1.65-1.85(m, 2H), 3.83(dd, 1H), 5.24(1H), 6.03(1H), 6.97(d, J=6.6Hz, 1H), 7.60-7.70(2H), 7.83(1H), 8.23(1H), 8.80-(1H), 8.97(1H), 9.61(d, J=8.3Hz, 1H).

$^1$H NMR (DMSO-$d_6$/ppm: high polarity isomer) δ 0.85(t, J=6.9Hz, 3H), 1.25-1.80(7H), 3.79(m, 1H), 5.24-(1H), 6.03(d, J=5.7Hz, 1H), 6.96(d, J=8.5Hz, 1H), 7.63(1H), 7.84(1H), 8.21(1H), 8.79(1H), 8.95(1H), 9.59(d, J=8.3Hz, 1H).

Example 44

i) Dissolved in a mixed solvent was 5.2g of 6-cyano-3,4-epoxy-3,4-dihydro-2,2-pentamethylene-2H-1-benzopyran, which is described in J. Med. Chem., 33,492 (1990), said mixed solvent consisting of 80 mℓ of a 28% aqueous ammonia and 90 mℓ of ethanol. The resultant aqueous solution was kept stirred for 14 hours. Then, 20 mℓ of aqueous ammonia was further added, followed by stirring for 23 hours and subsequently removing the solvent by evaporation to give 5.5g of trans-4-amino-6-cyano-3,4-dihydro-2,2-pentamethylen-2H-1-benzopyran-3-ol.

$^1$H NMR (CDCℓ$_3$/ppm) δ 1.10-2.10(m, 10H), 3.29(d, J=9.8Hz, 1H), 3.73(d, J=9.8Hz, 1H), 6.90(d, J=10.0Hz, 1H), 7.44(1H), 7.73(1H).

ii) Dissolved in 2.25 mℓ of ethanol were 392.5 mg of ethyl N-cyano-3-pyridinecarboximidate and 454.2 mg of trans-4-amino-6-cyano-3,4-dihydro-2,2-pentamethylene-2H-1-benzopyran-3-ol. The resultant solution was kept stirred for 23 hours, followed by removing the solvent by evaporation. Then, 233.5 mg of said imidate and 0.9 mℓ of ethanol were further added to the residue, followed by further stirring for 6.5 hours. Further, the solvent was removed by evaporation followed by crystallization from methanol to give 388.6 mg of N'-cyano-N-(trans-6-cyano-3,4-dihydro-3-hydroxy-2,2-pentamethylene-2H-1-benzopyran-4-yl)-3-pyridinecarboxamidine.

Melting point 231.8 - 215.8°C

$^1$H NMR (DMSO-$d_6$/ppm) δ 1.10-2.10(m, 10H), 3.69(m, 1H), 5.21(t, J=8.6Hz, 1H), 6.01(d, J=8.6Hz, 1H), 7.03(d, J=8.5Hz, 1H), 7.55-7.75(m, 1H), 7.88(1H), 8.21(1H), 8.79(1H), 8.94(1H), 9.59(d, J=8.3Hz, 1H).

Table 1 shows the chemical structures of the compounds synthesized in each of the Examples described above:

Table 1

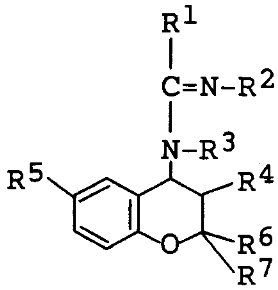

| Chemical Structures | | | | | | | |
|---|---|---|---|---|---|---|---|
| Example | $R^1$ | $R^2$ | $R^3$ | $R^4$ | $R^5$ | $R^6$ | $R^7$ |
| 1 | $CH_3$ | OH | H | OH | CN | $CH_3$ | $CH_3$ |
| 2 | $CH_3$ | $OCH_3$ | H | OH | CN | $CH_3$ | $CH_3$ |
| 3 | 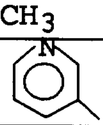 | CN | H | OH | CN | $CH_3$ | $CH_3$ |
| 4 | 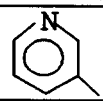 | $OCH_3$ | H | OH | CN | $CH_3$ | $CH_3$ |
| 5 | 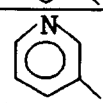 | OH | H | OH | CN | $CH_3$ | $CH_3$ |
| 6 | 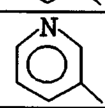 | CN | H | H | CN | $CH_3$ | $CH_3$ |
| 7 | 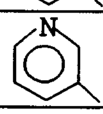 | OH | H | H | CN | $CH_3$ | $CH_3$ |
| 8 | $CH_3$ | $CH_2CN$ | $CH_3$ | H | CN | $CH_3$ | $CH_3$ |
| 9 | 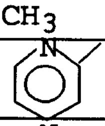 | CN | H | OH | CN | $CH_3$ | $CH_3$ |
| 10 | 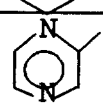 | CN | H | OH | CN | $CH_3$ | $CH_3$ |
| 11 | 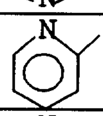 | $COCH_3$ | H | H | CN | $CH_3$ | $CH_3$ |
| 12 | 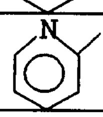 | $COCH_3$ | H | OH | CN | $CH_3$ | $CH_3$ |

| Chemical Structures | | | | | | | |
|---|---|---|---|---|---|---|---|
| Ex-sam-ple | $R^1$ | $R^2$ | $R^3$ | $R^4$ | $R^5$ | $R^6$ | $R^7$ |
| 13 | 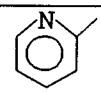 | $CO_2C_2H_5$ | H | H | CN | $CH_3$ | $CH_3$ |
| 14 | 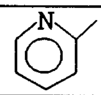 | $NO_2$ | H | OH | CN | $CH_3$ | $CH_3$ |
| 15 | 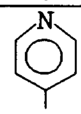 | CN | H | OH | CN | $CH_3$ | $CH_3$ |
| 16 | 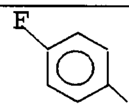 | CN | H | OH | CN | $CH_3$ | $CH_3$ |
| 17 (3S, 4R) | 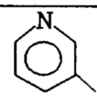 | CN | H | OH | CN | $CH_3$ | $CH_3$ |
| 18 | 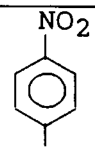 | $COCH_3$ | H | OH | CN | $CH_3$ | $CH_3$ |
| 19 | 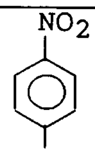 | $CO_2C_2H_5$ | H | OH | CN | $CH_3$ | $CH_3$ |
| 20 | $CH_3$ | OH | H | H | CN | $CH_3$ | $CH_3$ |
| 21 | $CH_3$ | $CO_2C_2H_5$ | H | OH | CN | $CH_3$ | $CH_3$ |
| 22 (3S, 4R) | 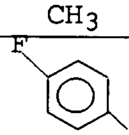 | CN | H | OH | CN | $CH_3$ | $CH_3$ |
| 23 | 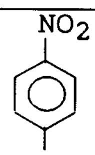 | CN | H | OH | CN | $CH_3$ | $CH_3$ |
| 24 (3S, 4R) | 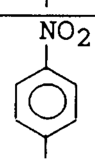 | CN | H | OH | CN | $CH_3$ | $CH_3$ |

(Continued)

| Chemical Structures | | | | | | | |
|---|---|---|---|---|---|---|---|
| Ex-sam-ple | $R^1$ | $R^2$ | $R^3$ | $R^4$ | $R^5$ | $R^6$ | $R^7$ |
| 25 | 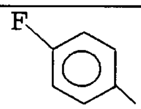 | $COCH_3$ | H | OH | CN | $CH_3$ | $CH_3$ |
| 26 | 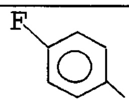 | $CO_2C_2H_5$ | H | OH | CN | $CH_3$ | $CH_3$ |
| 27 (3R, 4S) | 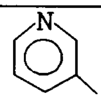 | CN | H | OH | CN | $CH_3$ | $CH_3$ |
| 28 | 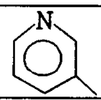 | CN | H | $OCOCH_3$ | CN | $CH_3$ | $CH_3$ |
| 29 | 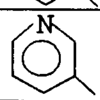 | CN | H | OH | F | $CH_3$ | $CH_3$ |
| 30 | 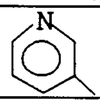 | CN | H | OH | $C_2H_5$ | $CH_3$ | $CH_3$ |
| 31 | 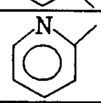 | CN | H | $ONO_2$ | CN | $CH_3$ | $CH_3$ |
| 32 | 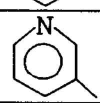 | CN | H | OH | $OCF_3$ | $CH_3$ | $CH_3$ |
| 33 | 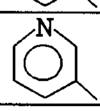 | CN | H | OH | $NO_2$ | $CH_3$ | $CH_3$ |
| 34 | 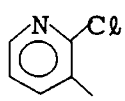 | CN | H | OH | CN | $CH_3$ | $CH_3$ |
| 35 | 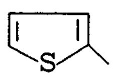 | CN | H | OH | CN | $CH_3$ | $CH_3$ |
| 36 | 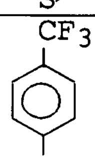 | CN | H | OH | CN | $CH_3$ | $CH_3$ |
| 37 | 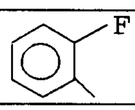 | CN | H | OH | CN | $CH_3$ | $CH_3$ |

(Continued)

| Chemical Structures | | | | | | | |
|---|---|---|---|---|---|---|---|
| Ex-sample | $R^1$ | $R^2$ | $R^3$ | $R^4$ | $R^5$ | $R^6$ | $R^7$ |
| 38 | 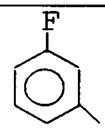 | CN | H | OH | CN | $CH_3$ | $CH_3$ |
| 39 | 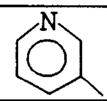 | CN | H | OH | Br | $CH_3$ | $CH_3$ |
| 40 | 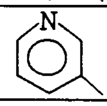 | CN | H | OH | $OCH_3$ | $CH_3$ | $CH_3$ |
| 41 | 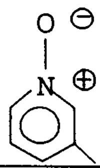 | CN | H | OH | CN | $CH_3$ | $CH_3$ |
| 42 | 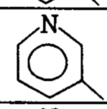 | CN | double bond between 3-and 4-position | | CN | $CH_3$ | $CH_3$ |
| 43 | 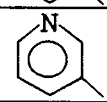 | CN | H | OH | CN | $C_3H_7$ | $CH_3$ |
| 44 | 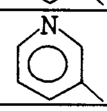 | CN | H | OH | CN | $-C_5H_{10}-$ | |

Let us describe the results of pharmacological evaluation of the compound of the present invention.

〈Blood Vessel Relaxing Action〉

Preparation of the extracted blood vessel sample and the experiment were conducted by the methods of Toda et al. described in J. Cardiovasc. Pharmacol., 7,1118 (1985). The aorta thoracica of a male Wister rat was used as a sample. To be more specific, a spiral piece was prepared by peeling endothelium about 1 cm long from the artery to provide the sample. The endothelium was peeled off by rubbing with a swab. A Krebs-Hensterite solution of 37°C saturated with 95% $O_2$ and 5% $CO_2$ was used as a nutrient solution. After application of 1g as suspending load to the sample, changes in tension were measured by rectigraph via an isometric transducer. After the aorta thoracica was contracted by phenylephrine ($10^{-6}$ M), a compound to be examined (an example compound) of $10^{-8}$ to $10^{-5}$ M was cumulatively added to determine the relaxing action of the compound quantitatively. The relaxing effect of the example compound was determined in terms of the concentration $IC_{50}$ of the compound required for inhibiting 50% of the contraction achieved by phenylephrine ($10^{-6}$ M). Lema Kalin was used as reference compound A. On the other hand, reference compound B was provided by N'-cyano-N-(trans-6-cyano-3,4-dihydro-2,2-dimethyl-3-hydroxy-2H-1-benzopyran-4-yl)-benzamidine. Table 2 shows the results.

Table 2

| Example | 3 | 14 | 16 | Reference Compound A | Reference Compound B |
|---|---|---|---|---|---|
| $IC_{50}$ (μm) | 0.6 | 1.0 | 0.06 | 0.4 | 1.0 |

〈Action on the Coronary Blood Flow Rate in Dog〉

Dogs with ether sex were anesthetized with pentobarbital (30 mg/kg,intravenous administration), followed by intravenous heparin administration (500 U/kg) to the dogs under artificial respiration. Then, according to Yago's method described in Folia pharmacol. japon., 57,380 (1961), the left coronary artery was perfused with the right femoral arterial blood so as to measure the blood perfusion amount with an electromagnetic blood flow meter. The example compound was administered into the coronary artery by using a micro syringe just before a stainless steel cannula inserted into the coronary artery. The increase in the coronary blood flow rate caused by the example compound was determined by standardizing the reactivity among the specimen subject, with the reaction caused by the niphedipine administration (1 $\mu$g) into the coronary artery of the specimen subject being set at 100%. To be more specific, the intensity of the action due to the example compound was determined in terms of $ED_{50}$ required for increasing the coronary blood flow rate by 50% on the basis of the reaction caused by niphedipine administered into the coronary artery in an amount of 1 $\mu$g. Further, the duration of the effect produced by the example compound was determined in terms of the time (minutes) during which the compound produced at least 50% of efficacy on the basis that the efficacy of the compound in the $ED_{50}$ dosage noted above was set at 100%. Table 3 shows the results.

Table 3

| Example | 3 | 16 | Reference Compound A | Reference Compound B |
|---|---|---|---|---|
| ED 50(g) | 1.9 | 1.5 | 1.1 | 4.2 |
| Durationng (minuted) | 4.0 | 2.5 | 2.5 | 1.9 |

〈Bloodless Blood Pressure Measurement〉

The tail artery systolic pressure and heart rate of a non-anesthetic rat were measured with a bloodless automatic blood pressure meter by the method of Y. Iemori et al. described in Japan Heart J., 15,209 (1974), using a spontaneously hypertensive rat (male, age of 16 weeks old). Before measuring the blood pressure, the rat was warmed for about 10 minutes within a warming box set at 37°C. Then, the rat was fixed to a rat holder so as to measure the blood pressure at 37°C. The systolic pressure and the heart rate were measured before the administration of the example compound and 2, 4, 6, 8, 12 and 24 hours after the administration. The compound, i.e., 0.5% suspension in sodium carboxymethyl cellulose solution, was orally administered to the rat. The effects on the blood pressure reduction and on the heart rate were evaluated in terms of the change (%) relative to the values before the administration. Table 4 shows the results.

Table 4

| Example | Time(hour) | 2 | 4 | 6 | 8 | 12 | 24 |
|---|---|---|---|---|---|---|---|
| 3 (3mg/kg) | Blood Pressue fall (%) | -17 | -20 | -16 | -14 | - 9 | - 9 |
| | Heart Rate(%) | 10 | 9 | 5 | 1 | - 2 | - 8 |
| 14 (3mg/kg) | Blood Pressue fall (%) | -15 | -22 | -29 | -35 | -35 | -33 |
| | Heart Rate(%) | 9 | 25 | 28 | 28 | 30 | 23 |
| 14 (1mg/kg) | Blood Pressue fall (%) | - 2 | - 5 | -15 | -17 | -23 | -23 |
| | Heart Rate(%) | 5 | 6 | 13 | 14 | 17 | 20 |
| 16 (0.3mg/kg) | Blood Pressue fall (%) | -37 | -41 | -44 | -49 | -42 | -27 |
| | Heart Rate(%) | 38 | 34 | 32 | 31 | 30 | 11 |
| Reference Compound A (0.3mg/kg) | Blood Pressue fall (%) | -27 | -13 | -10 | -11 | - 6 | - 5 |
| | Heart Rate(%) | 18 | 5 | 2 | 1 | - 6 | - 7 |

## Claims

1. A novel benzopyran derivative represented by general formula [I] given below and a pharmaceutically acceptable salt thereof:

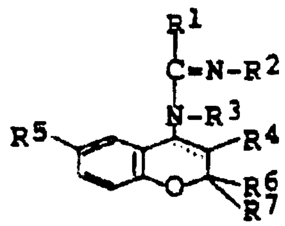

(I)

where ⋯ denotes a single bond or a double bond;
$R^1$ is (a) a lower alkyl group which may be substituted with at least one halogen atom, at least one

nitro group, at least one lower alkoxycarbonyl group or at least one cyano group, (b) a cycloalkyl group having 3 to 7 carbon atoms, (c) an aryl group which may be substituted with at least one substituent selected from the group consisting of et least one halogen atom, at least one lower alkyl group, at least one halogen-substituted lower alkyl group, at least one nitro group, at least one lower alkoxycarbonyl group, at least one cyano group, at least one lower alkylsulfonyl group, at least one aminosulfonyl group, at least one acylamino group, at least one lower alkylsulfonylamino group, at least one halogen-substituted acyl group and at least one acyl group, or an aralkyl group which may be substituted with at least one said substituent, (d) a hetero aryl group which may be substituted with at least one halogen atom, at least one lower alkyl group, at least one nitro group, at least one lower alkoxycarbonyl group and at least one cyano group, or (e) a saturated hetero ring group represented by the formula given below, which has 3 to 6 carbon atoms,

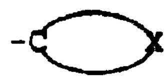

where X is O, S or N-$R^8$, $R^8$ being a lower alkyl or acyl group,

$R^2$ is a hydroxyl group, a lower alkoxy group, a cyano group, a nitro group, a cyanomethyl group or a group -A-$R^9$ or

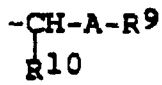

where A is a carbonyl group or a sulfonyl group, $R^9$ is a lower alkyl group which may be substituted with at least one halogen atom, a lower alkoxy group, or an amino group which may be substituted with a lower alkyl group, end $R^{10}$ is a hydrogen atom or a lower alkyl group;

$R^3$ is a hydrogen atom or a lower alkyl group;

$R^4$ is a hydrogen atom, a hydroxyl group, a nitroxy group or an acetoxy group;

$R^5$ is a lower alkyl group, a lower alkoxy group which may be substituted with at least one halogen atom, a cyano group, a nitro group, an acyl group or a halogen atom; and

$R^6$ and $R^7$, which may be the same or different, are lower alkyl groups or collectively form an alkylene group having 4 to 6 carbon atoms;

with the proviso that where $R^2$ represents a cyano group, $R^1$ is not an unsubstituted lower alkyl group, an unsubstituted aralkyl group, an unsubstituted aryl group nor a lower alkyl-substituted aryl group.

**2.** The novel benzopyran derivative or a phamaceutically acceptable salt thereof according to claim 1, wherein said $R^2$ is a hydroxyl group, a lower alkoxy group, a nitro group, a cyanomethyl group or a group -A-$R^9$ or

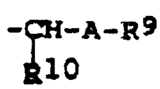

where $R^9$ and $R^{10}$ are as defined in claim 1.

**3.** The novel benzopyran derivative or a phamaceutically acceptable salt thereof according to claim 1, wherein said $R^1$ is a phenyl group which may be substituted with at least one substituent selected from the group consisting of a halogen atom, a halogen-substituted lower alkyl group, a nitro group, a lower alkoxycarbonyl group, a cyano group, a lower alkylsulfonyl group, an aminosulfonyl group, a halogen-substituted acyl group and an acyl group; or a hetero aryl group selected from the group consisting of a pyridinyl group, a pyradinyl group, a pyrimidinyl group, a pyridadinyl group, a furanyl group, a thienyl group and a pyrrolyl group.

**4.** The novel benzopyran derivative or a phamaceutically acceptable salt thereof according to claim 3, wherein said $R^2$ is a cyano group.

5. A potassium channel activating agent, which contains the novel benzopyran derivative or a pharmaceutically acceptable salt thereof recited in claim 1.

# INTERNATIONAL SEARCH REPORT

International Application No PCT/JP92/00538

**I. CLASSIFICATION OF SUBJECT MATTER** (if several classification symbols apply, indicate all) [6]

According to International Patent Classification (IPC) or to both National Classification and IPC

Int. Cl[5] C07D311/68, C07D405/12, C07D409/12//A61K31/35(C07D405/12, C07D213:00, C07D311:00)(C07D409/12, C07D311:00, C07D333:00)

**II. FIELDS SEARCHED**

Minimum Documentation Searched [7]

| Classification System | Classification Symbols |
|---|---|
| IPC | C07D311/68, C07D405/12, C07D409/12, (C07D405/12, C07D213:00, C07D311:00)(C07D409/12, C07D311:00, C07D333:00) |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| EX | JP, A, 3-279378 (Kaken Pharmaceutical Co., Ltd.), December 10, 1991 (10. 12. 91), (Family: none) | 1-5 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| July 15, 1992 (15. 07. 92) | August 4, 1992 (04. 08. 92) |
| **International Searching Authority** | **Signature of Authorized Officer** |
| Japanese Patent Office | |